# EUROPEAN PATENT SPECIFICATION

(11) **EP 3 104 786 B1**
(45) Date of publication and mention of the grant of the patent: **09.09.2020**
(21) Application number: 15705290.3
(22) Date of filing: 16.02.2015
(51) Int. Cl.: A61B 17/04

(54) **SURGICAL DEVICE**
CHIRURGISCHE VORRICHTUNG
DISPOSITIF CHIRURGICAL

(30) Priority: 14.02.2014 GB 201402681; 19.06.2014 GB 201410980
(43) Date of publication of application: 21.12.2016
(73) Proprietor: NeoSurgical Limited, Dublin 24 (IE)
(72) Inventor: RUSSELL, Barry, Dublin 24 (IE); KEATING, Ronan, Dublin 24 (IE); RABBITTE, Gerard, Dublin 24 (IE)
(74) Representative: Hanna Moore + Curley
(86) International application number: PCT/EP2015/053243
(87) International publication number: WO 2015/121478

(56) References cited:
- US-A1- 2002 188 301
- US-A1- 2006 190 041
- US-A1- 2010 113 873
- US-A1- 2011 160 766
- US-A1- 2011 245 846
- US-A1- 2012 059 414
- US-A1- 2013 324 926

## Description

### Field of the Invention

The present invention relates to a surgical device and particularly to a suture delivery system. In one configuration it relates to a suture delivery system which operably provides for deployment of an anchoring system which is usefully employed in laparoscopic, robotic, or general surgery. In another configuration it relates to a suture delivery system which operably provides a closure system which is usefully employed in laparoscopic, robotic, or general surgical procedures.

### Background

There are difficulties sometimes associated with the closure of wound sites, for example, in laparoscopic procedures. There are difficulties in particular in finding the fascia layer through which a suture must be passed to ensure adequate port site closure.

With deeper port sites, such as with an obese patient, it is often more difficult for the surgeon to gain deep access to the fascial layer to securely place a suture therein. In certain instances it may be necessary to cut open the wound to accurately place a suture fixation on the inner fascia layer.

The consequences of inadequate closure may be serious. For example, the patient may be subject to an early or late onset hernia, bowel stricture and/or bleeding from the port site. All of these complications have varying associated morbidities up to and including fatalities in serious undetected bowel strictures. The rate of port site herniation is widely published to be up to 3% for the normal population and double this for the obese cohort.

There are therefore a number of problems with current methods of trocar port site closure that need to be addressed, particularly for the obese patient. These and other problems are also found in non-laparoscopic surgical techniques where there is a desire to deliver suture to a wound site.

Many closure devices use an angled trajectory to deliver suture or anchors through the abdominal wall and use the outer surface of the abdominal wall as a datum. The problem with this approach is that the thickness of the abdominal wall can vary from patient to patient, and from port to port in the same patient. This leads to a variation in deployment accuracy.

Another issue with such closure methods is that there is invariably a sharp tip being delivered through the abdominal wall, towards the patient, which once through has the potential to damage internal organs.

US 2013/324926 describes a device which is designed to facilitate gastrointestinal bypass and includes a removable anchor.

The device disclosed herein attempts to provide a solution to these problems.

### Summary

Accordingly, a first embodiment of the application provides a device per the independent claim 1. Advantageous embodiments are provided in the dependent claims.

### Brief Description of the Drawings

The present invention will now be described with reference to the accompanying drawings in which:
Figures 1A to 1B shows examples of a closure of a port site with in accordance with the present teaching;
Figures 2A through 2G show examples of a device that may be provided in accordance with the present teaching;
Figures 3A and 3D show examples of a device that may be provided in accordance with the present teaching;
Figures 4A to 4G show examples of a device that may be provided in accordance with the present teaching;
Figures 5A to 5D show examples of a device that may be provided in accordance with the present teaching;
Figures 6A to 6C show examples of a device that may be provided in accordance with the present teaching;
Figures 7A to 6G show examples of a device that may be provided in accordance with the present teaching;
Figures 8A to 8D show examples of a device that may be provided in accordance with the present teaching;
Figures 9A to 9F show examples of a device that may be provided in accordance with the present teaching;
Figures 10A-10C show examples of a device that may be provided in accordance with the present teaching;
Figures 11A-11C show examples of a device that may be provided in accordance with the present teaching;
Figures 12A to 12I show examples of a device that may be provided in accordance with the present teaching;
Figures 13A to 13C show examples of a device that may be provided in accordance with the present teaching; and
Figures 14A to 14F show examples of a device that may be provided in accordance with the present teaching.
Figures 15A to 15B show examples of a device pre deployment may be provided in accordance with the present teaching.
Figures 16A to 16B show examples of a device post deployment that may be provided in accordance with the present teaching.
Figures 17A to 17EB show examples of a device that may be provided in accordance with the present teaching.
Figures 18A to 18B show examples of a device that may be provided in accordance with the present teaching.
Figures 19A to 19B show examples of a device that may be provided in accordance with the present teaching.
Figures 20A to 20B show examples of a device that may be provided in accordance with the present teaching.

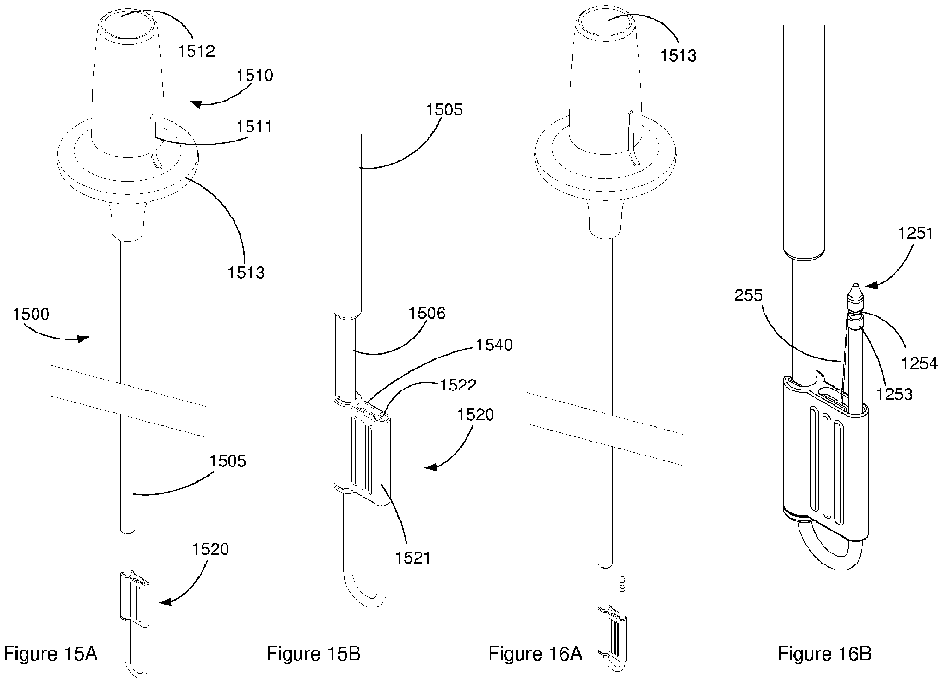

Figures 15A to 15B show examples of a device that may be provided in accordance with the present teaching.

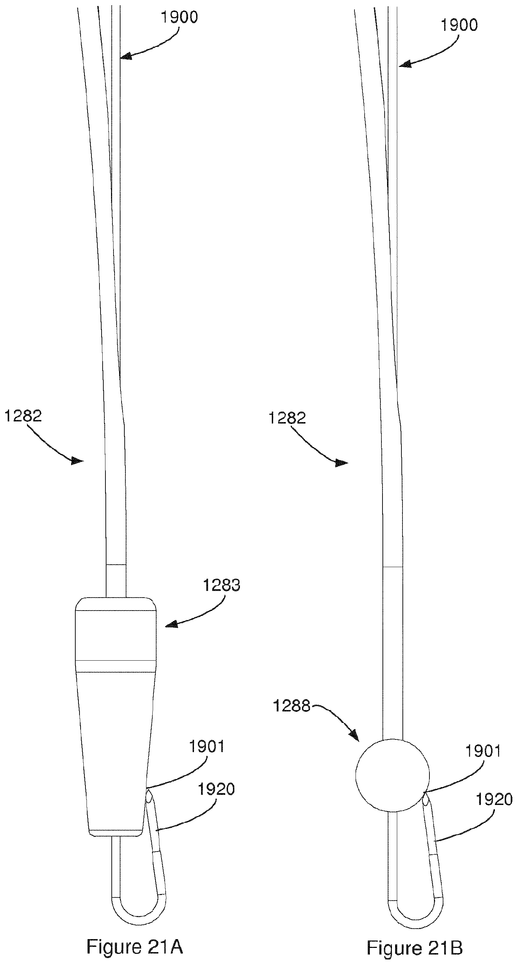

Figures 21A to 21B show examples of a device that may be provided in accordance with the present teaching.
Figures 15A to 15B show examples of a device that may be provided in accordance with the present teaching

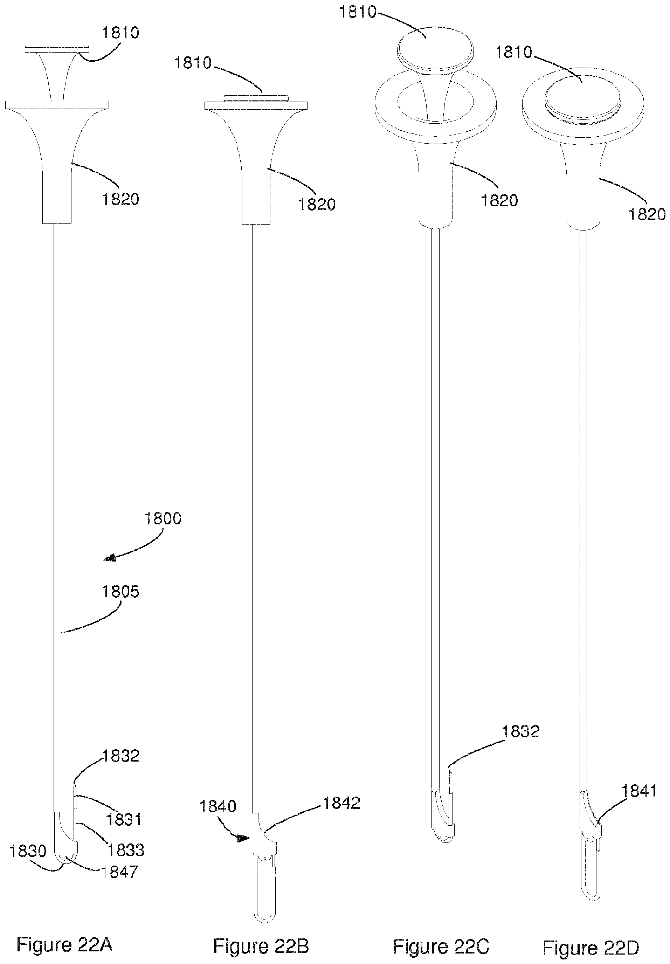

Figures 22A to 22D show examples of a device that may be provided in accordance with the present teaching.

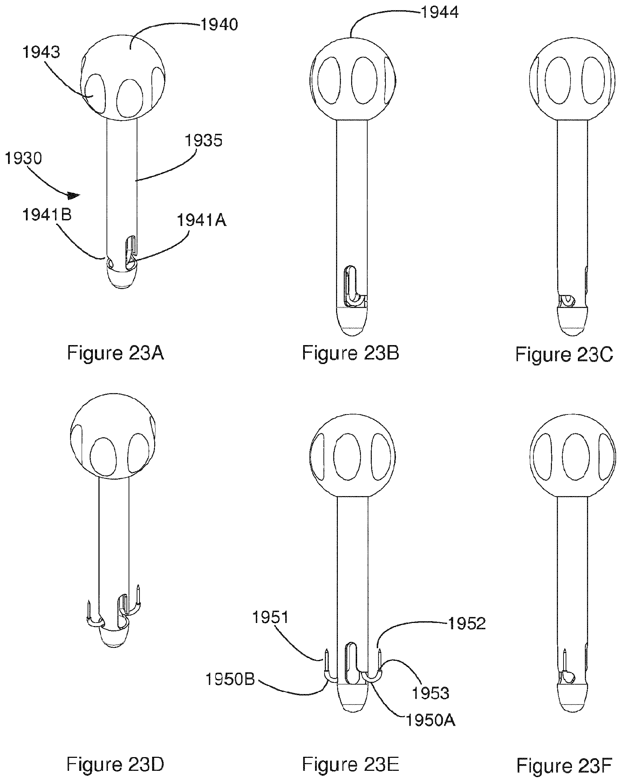

Figures 23A to 23F show examples of a device that may be provided in accordance with the present teaching.

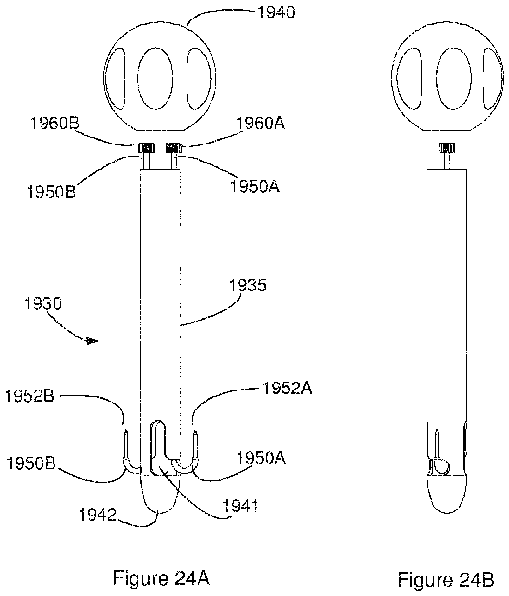

Figures 24A to 24B show examples of a device that may be provided in accordance with the present teaching.

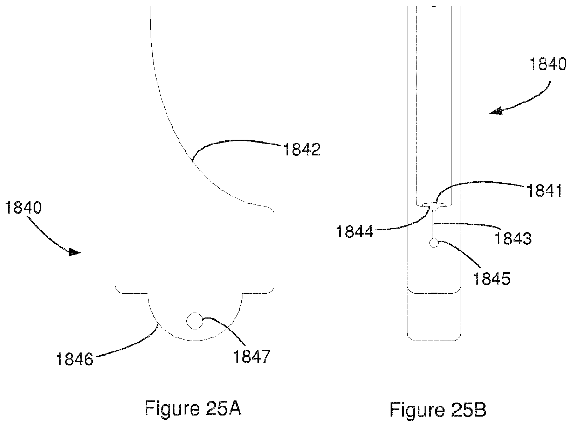

Figures 25A to 25B show examples of a shield portion of a device that may be provided in accordance with the present teaching.

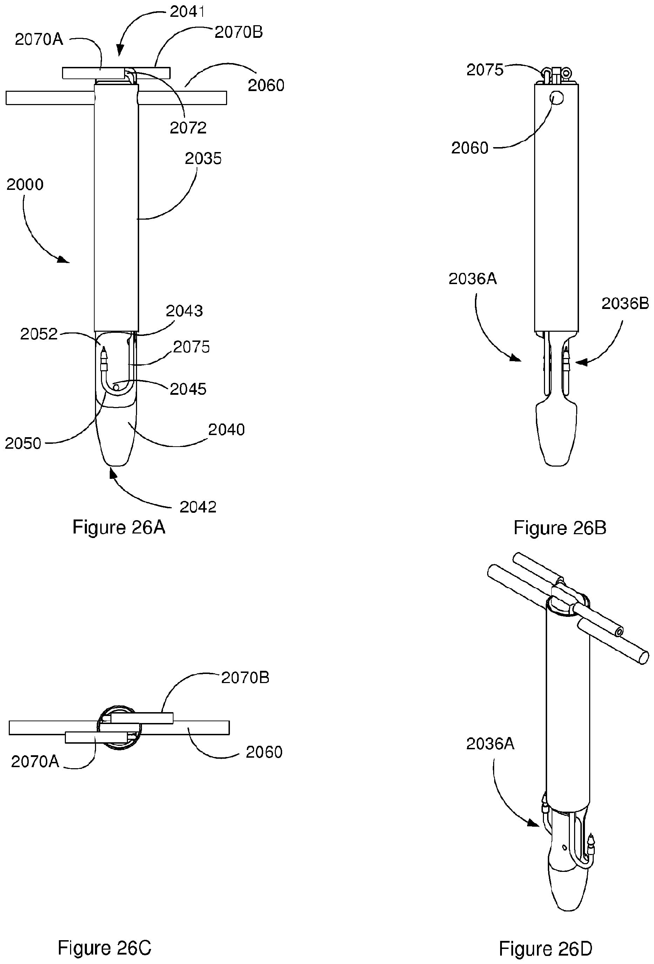

Figures 26A to 26F show examples of a shield portion of a device that may be provided in accordance with the present teaching

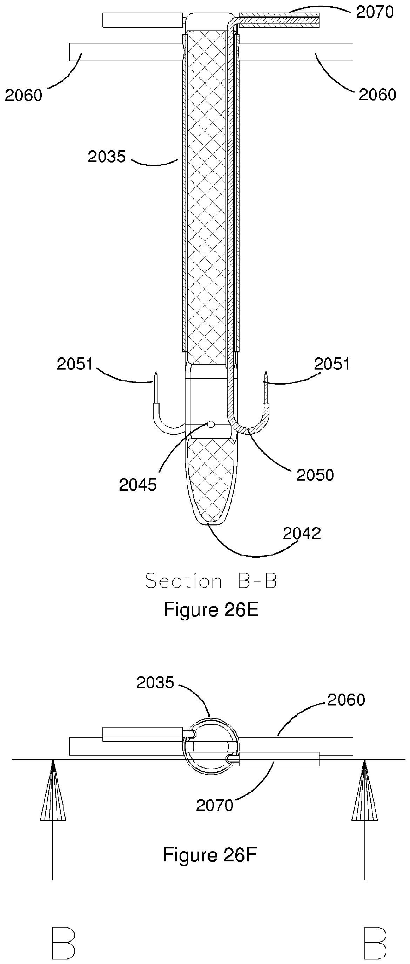

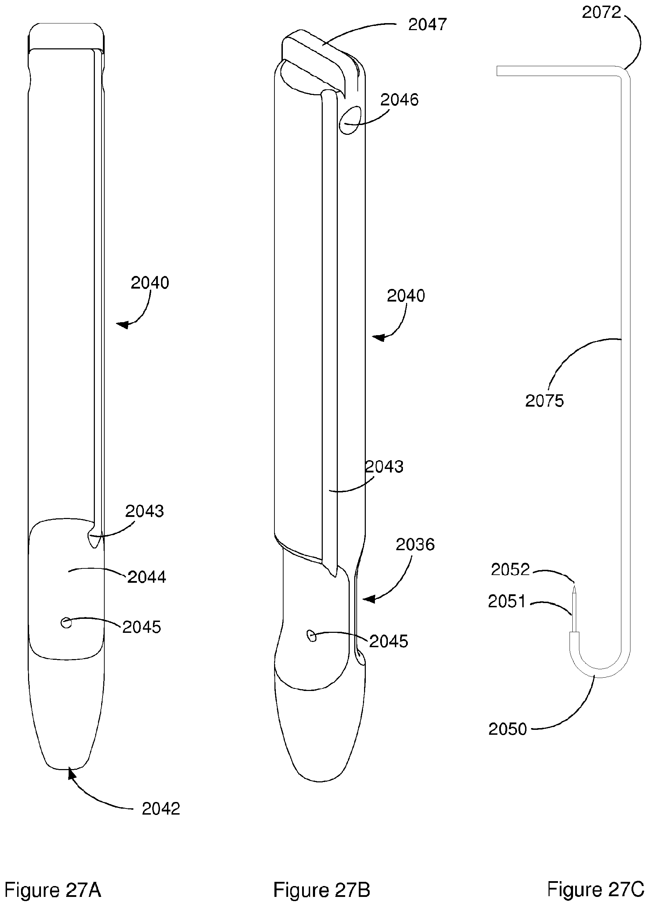

Figures 27A to 27C show components of the device of Figure 26 in an unassembled state.

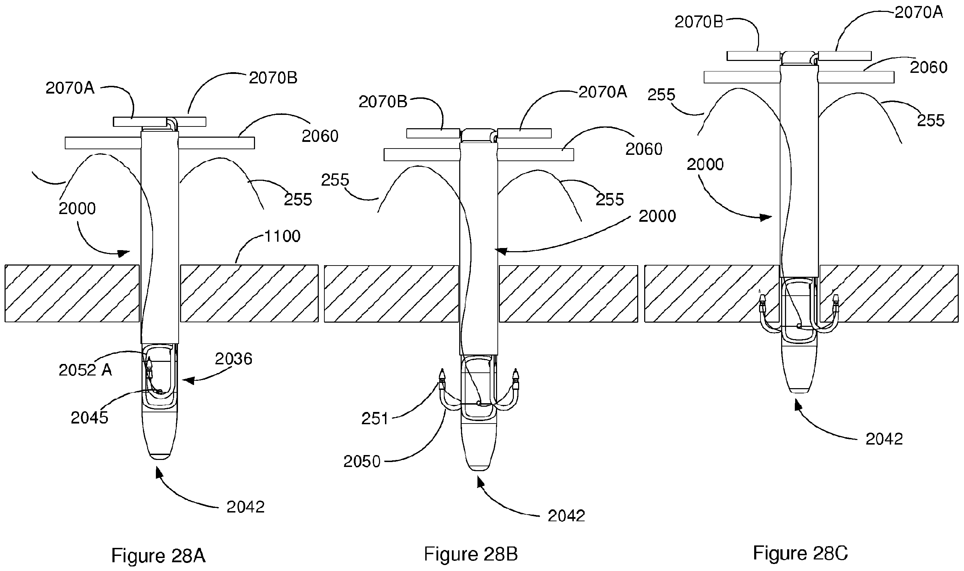

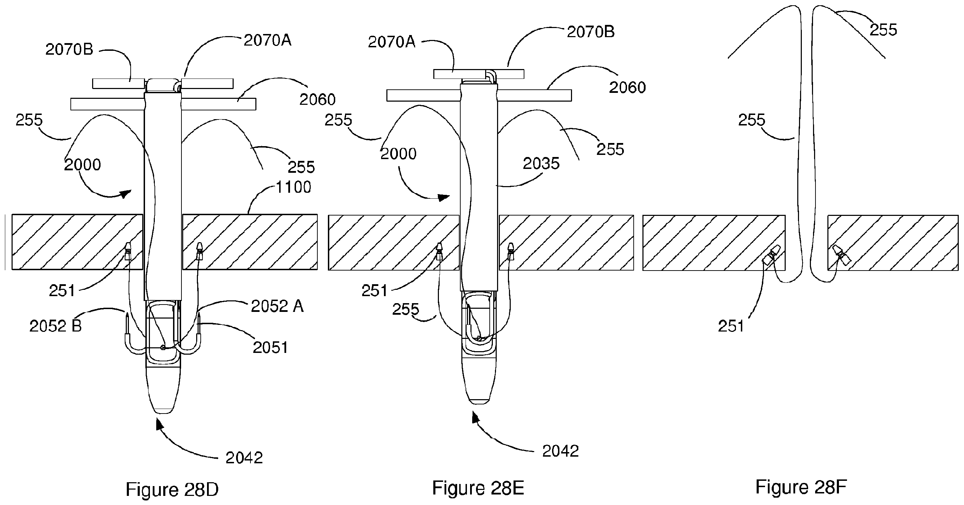

Figures 28A to 28E show in schematic form use of the device of Figure 26.

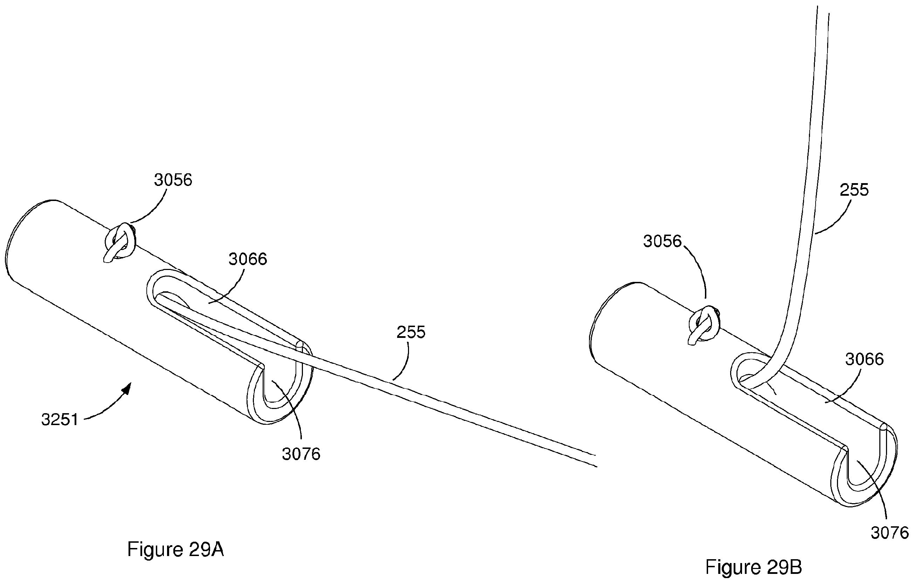

Figures 29A and 29B show examples of an anchor that may be provided in accordance with the present teaching.

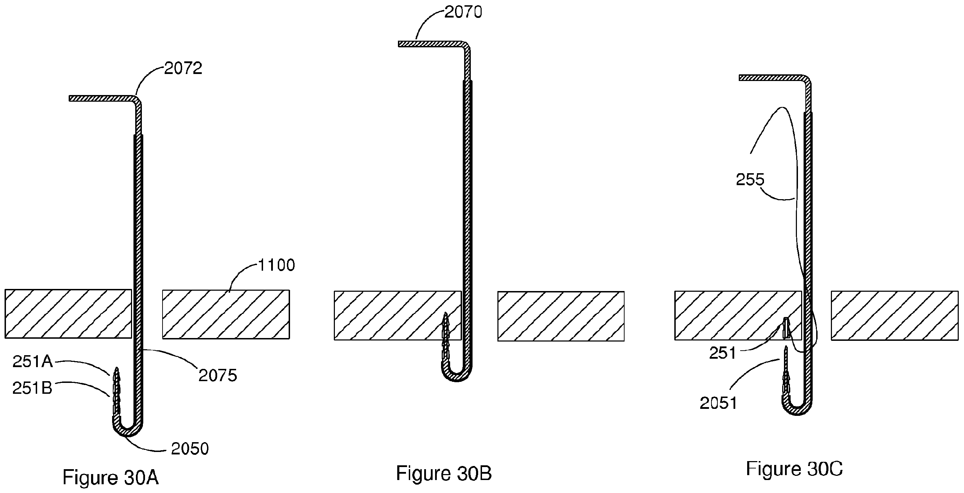

Figures 30A to 30C show examples of an anchor delivery device in accordance with the present teaching.

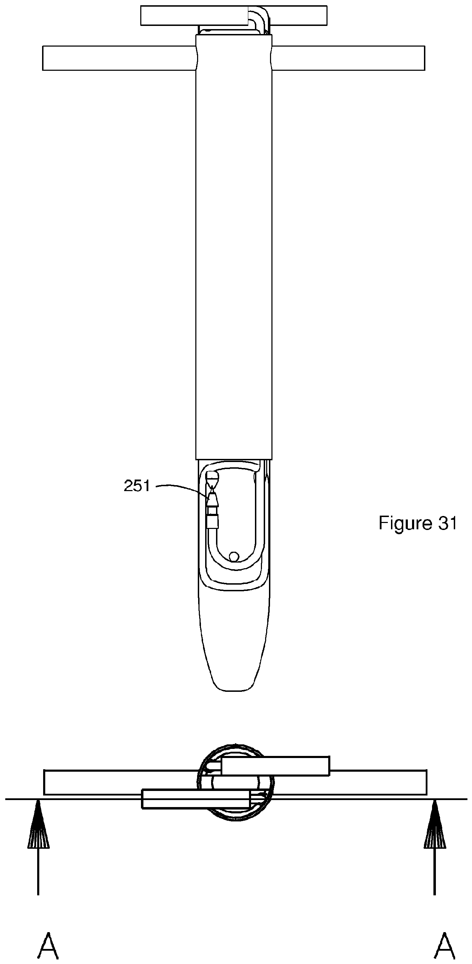

Figure 31 shows a modification to the device of Figure 26 to incorporate a plurality of anchors.

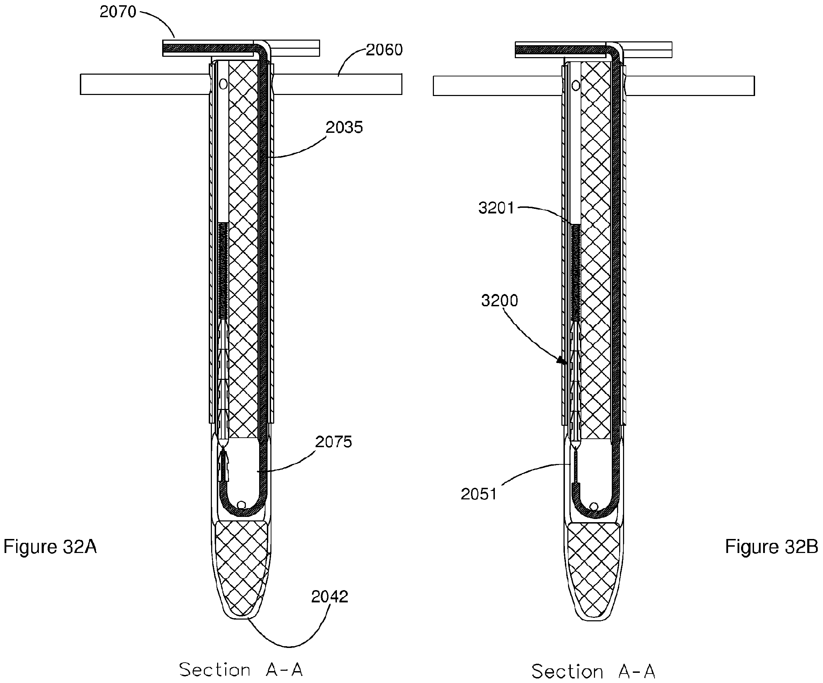

Figures 32A to 32E are sections through the device of Figure 31 showing the additional anchors provided in a stored condition within the housing.

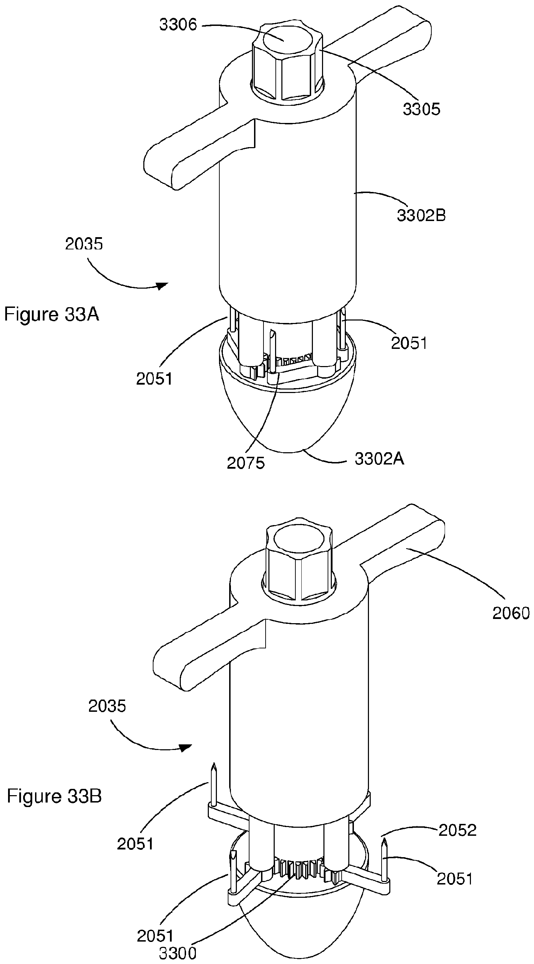

Figures 33A and 33B show an example of a device provided in accordance with the present teaching.

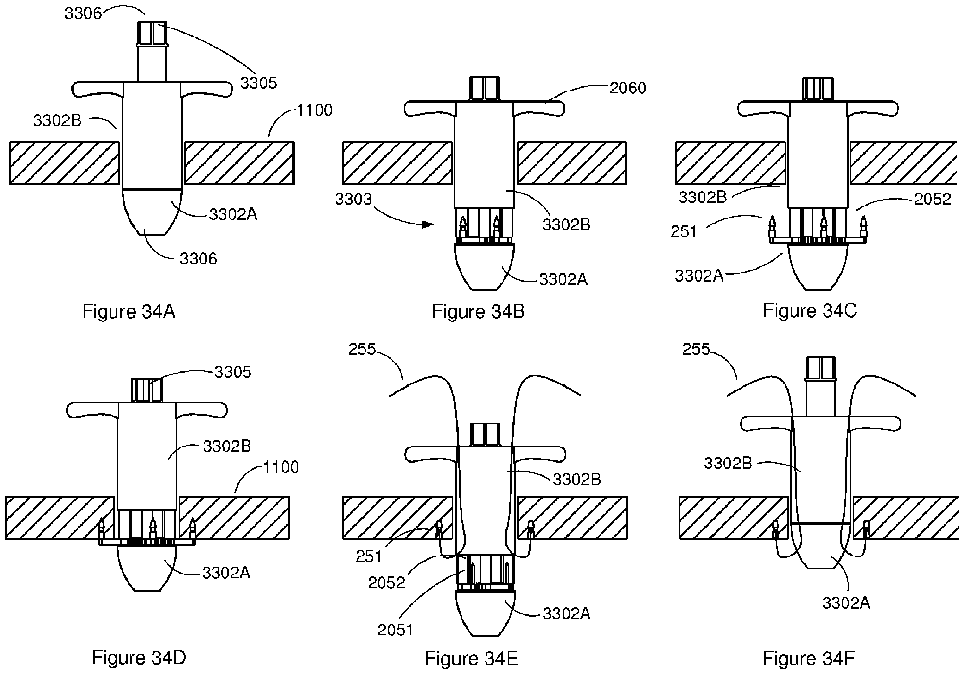

Figure 34A to 34F shows example of how the device of Figure 33 can be used to deploy anchors.

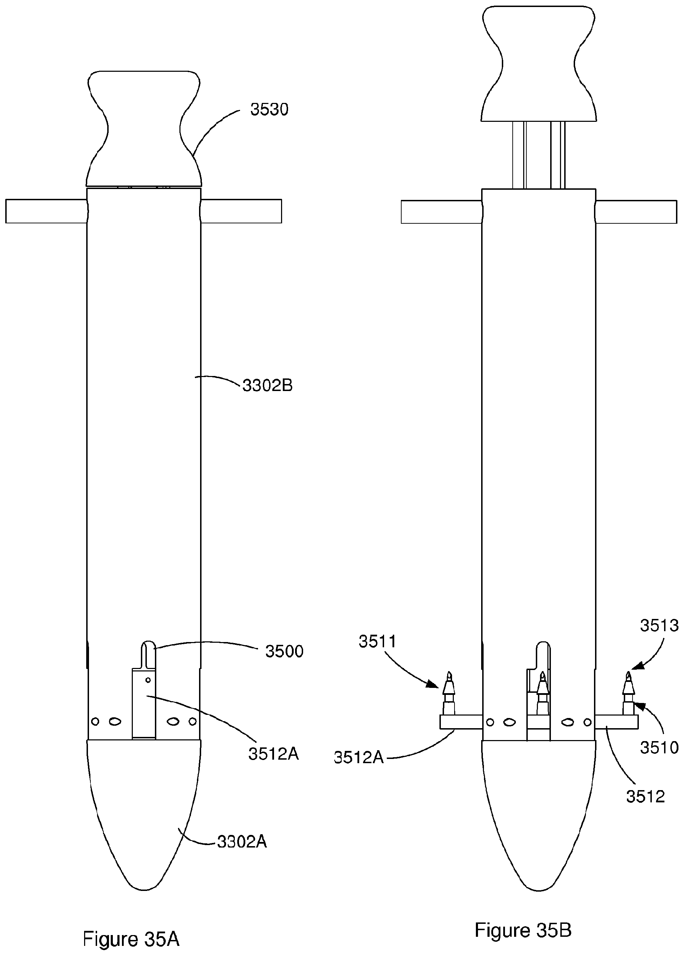

Figures 35A to 35C are examples of a modified version of the device of Figure 33.

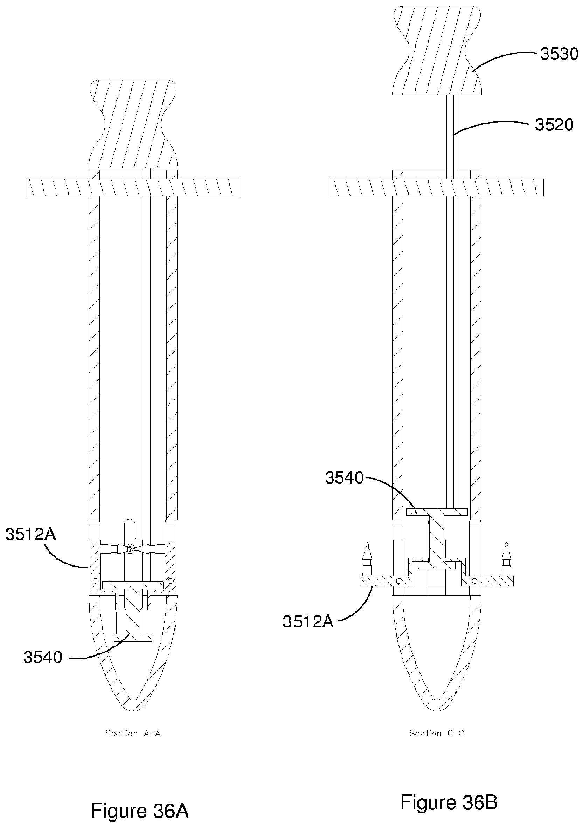

Figure 36A and 36B show another example of a device in accordance with the present teaching.

### Detailed Description of the Drawings

The teaching of the present invention will now be described with reference to exemplary embodiments thereof which are provided to assist with an understanding of the present teaching and are not to be construed as limiting in any way. It will be appreciated that modifications can be made to the exemplary arrangements which follow without departing from the spirit or scope which is only to be limited insofar as is deemed necessary in the light of the appended claims.

Within the context of the present teaching a suture delivery system advantageously allows for the delivery of suture within an abdominal cavity of the patient. It will be appreciated that the following discussion regarding the specifics of the abdominal cavity and abdominal wall should not be construed as limiting in that a system provided in accordance with the present teaching may be used with other types of tissue including but not limited to organs, bones or the like. The use of a suture delivery system per the present teaching can be used for one or more of anchoring laparoscopic surgical equipment, assisting in the moving of internal organs to allow a surgeon access to a surgical site, or closure of a wound post completion of a surgical procedure. In such a latter configuration, where the suture is coupled to an anchor, as the suture is passed through the abdominal wall and is held within the wall by the anchors that will remain deployed within the abdominal cavity, a subsequent tightening of the sutures will cause the sides of the incision, or defect in the abdominal cavity to be brought together to close the wound. The adoption of such a technique will advantageously require the use of bioabsorbable anchors, as the anchors will remain within the abdominal cavity during the healing process prior to their ultimate disintegration.

In accordance with the present invention an anchor is coupled to a suture. The anchor may then be delivered to the surgical site through cooperation of the anchor with a delivery tool. The delivery tool engages with the anchor and is then used to deliver the anchor and its associated suture through to the abdominal cavity. The anchor lumen, once delivered into tissue is hollow, and can advantageously promote tissue in growth which strengthens the hold of the anchor on surrounding tissue.

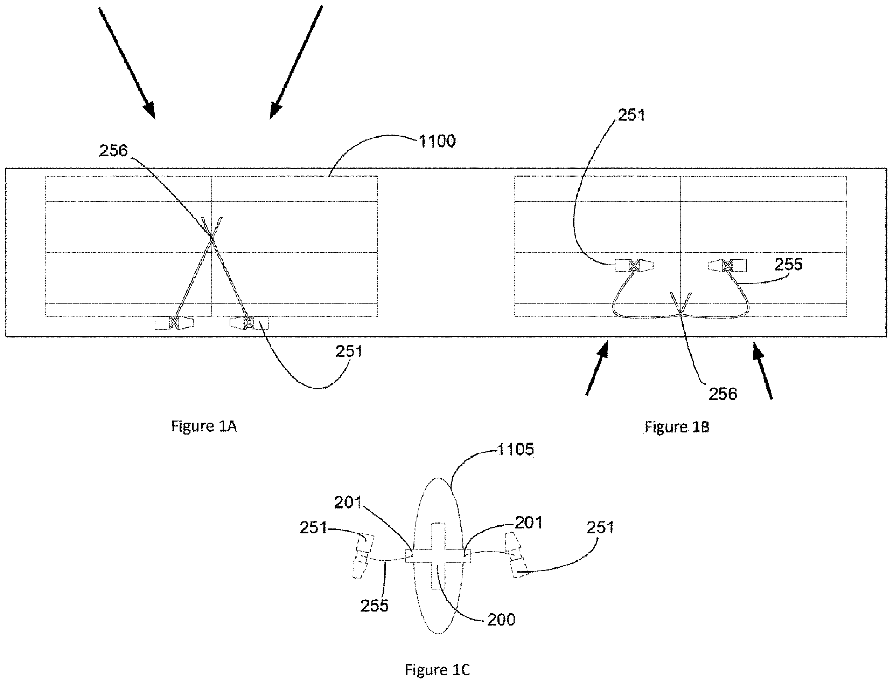

Figure 1A illustrates a closure of a defect where the anchor 251 has been placed through the fascia 1100 from outside the abdominal space. The angle of placement is shown by the arrows in figure 1A. This is a conventional technique for placing anchors 251 using a known anchor delivery device with the trajectory of the anchors indicated by the arrows.

The device of the present invention presents the anchor through the fascia 1100 from inside the abdominal space as shown in Figure 1B, following a trajectory illustrated by the arrows. The suture 255 is then knotted 256 to create the closure. Alternately suture strands could be conjoined to a cinch, not shown. The cinch could be slid down the suture to pull the anchor close together effecting closure of the defect. The cinch could be designed to be biodegradable such that it does not need to be removed once it is paced in the wound. Additionally the cinch could feature a geometry which is designed to prevent ingress of tissue into the defect area. Alternately a bioabsorbable mesh could be incorporated onto the suture such that when the anchors 251 are drawn together the mesh covers the area of the defect.

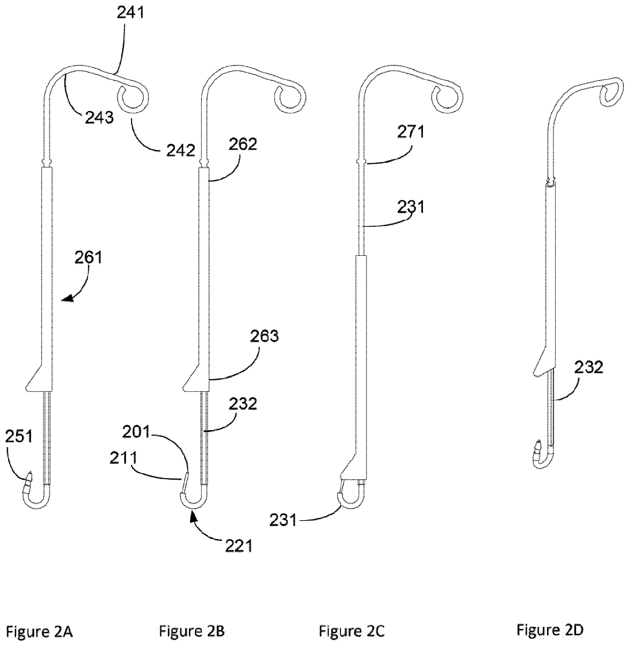

A first embodiment of a device for delivering anchors and suture in the form outlined in Fig 1B is illustrated in Fig 2A. The principle element of the design comprise of a needle portion 201, an anchor receiving portion 211, a curved portion which could be dimensioned in certain examples to define a hook or a u-bend portion, a shaft portion 231 and a proximal indicator 241 to enable the user to envisage the orientation of the needle. The needle portion 201 could be formed from a wire with a diameter which is smaller than the receiving inner diameter of an anchor 251. This wire could then be inserted into a straight hypo-tube and be formed in a u shape, which retains the wire. Alternately a larger diameter wire could be used in place of the hypo tube, and a step down portion could be ground to create the anchor receiving portion and the needle tip. The proximal indicator 241 could be a form on the wire 243 which is orientated in the same plane as the u-bend portion 221. A proximal end of the wire proximal indicator 241 may be formed into a loop 242, which has the advantage of minimizing the risk of stab type injuries to the user of the device, or the potential for ripping a surgical glove and compromising the sterile field. It will be appreciated that other means for minimising the risk of stab injuries could be used such as a cover over the exposed wire of the proximal indicator 241. Additionally the proximal indicator can function as a handle, giving the user something with which to apply upward pressure to the distal end of the device, or rotation of the device.

This embodiment also features a shield 261. This shield could be formed from a heat shrink material, heated over a form with the desired shape, or be a moulded component. The shield features a proximal portion 262 where the user can push or retract the shield and a distal portion 263 which is designed to cover the needle and/or anchor. The shield can be advanced to protect the tip as illustrated in Figure 2C. It will be understood that this movement is parallel to a longitudinal axis of the shaft 231 such that the anchor can be exposed or housed within the shield dependent on the movement of the shield along the shaft 231. The location of the anchor within the housing formed by the shield could be usefully employed when the device is being placed in a trocar or through a defect to prevent the anchor from catching on an internal feature of the trocar or the tissue in the defect and prematurely deploying. The shield could also be useful on retrieval of the device, as there is a risk the needle 201 would catch on the trocar seal or tissue on removal. The shield 261 prevents such incidents as it completely covers the needle tip 201. To ensure that the shield 261 is as small a profile as possible and covers the target area each time, the shaft 231 could feature a flat portion 232. This corresponds to a similar geometry on the inside of the shield, which in cooperation prevents the shield from rotating relative to the u-bend portion i.e., around the shaft portion 231. While it is illustrated as such a person skilled in the art will appreciate that there are many ways to prevent such rotation and the illustrated configuration is not intended to be limiting. The distal or forward movement of the shield is prevented by the distal portion 263 of the shield contacting the needle/anchor. Proximal movement of the shaft could be limited by a crimped portion 271 of the shaft 231 as illustrated in Fig 2C.

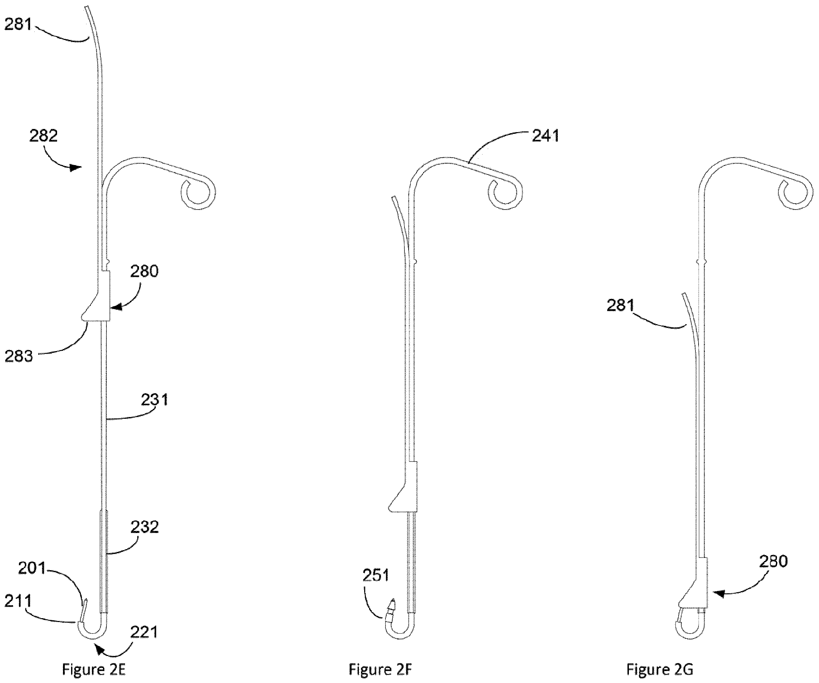

To use the device of Figure 2 the user would first have the shield 261 deployed to protect the anchor as shown in Figure 2C. The anchor 251 may feature a suture, not shown, which is either held at the proximal end of the device or cinched in a feature at the proximal end of the device. The device is used as follows:
a. The device is passed through a trocar until the u-bend portion is in the inner abdominal space;
b. The user would then retract the shield, exposing the anchor/needle;
c. The trocar is retracted until the edge of the trocar is in line with the inner abdominal wall;
d. The device is then angled to maximise the fascial recruitment of the device and the user pulls up once satisfied with the anchor/needle entry point. The anchor is delivered into the fascia;
e. The user pushes down on the device to release the anchor;
f. The user advances the shield to protect the needle, before removing the device through the trocar;
g. A second anchor can be delivered as previously described with reference to Figure 1B, at 180° to the first. The trocar may then be removed;
h. The free ends of the suture, which are outside the body can be knotted and the knot can be pushed down until adequate approximation is achieved.

A similar method can be used for delivery of anchors through a defect where no trocar is used. An advantage of the approach entailed in the design of the device from Fig 2 is that a sharp tip (such as the needle portion 201 or anchor 251 held thereon) is not forward facing or facing in the direction of the patient, so that organ injury is less likely as the device is advanced through a trocar of an abdominal wall.

It will be appreciated by those skilled in the art that the geometry of the u-bend portion 221 can influence many aspects of the operation of the device. The distance from the proximal end of the anchor receiving portion 211 to the distal tip of the u-bend portion 221 determines the depth to which the anchor can be pulled up into or imbedded in the abdominal wall. The shape of the u-bend portion 221 can influence the distance the tip 201 is from the shaft. This is important in determining the maximum fascial recruitment and would advantageously be in the range of 5-7mm on each side of the defect. Different applications would require different fascial recruitment depending on the tissue type and quality being approximated. Similarly, the angle of the needle 201 to the main shaft 231 will have an influence on the area necessary to protect the needle tip. The shape of the u-bend portion 221, coupled with shaft diameter is also important in determining the trocar size. The device is compatible with and will be optimally designed for size 10 Trocars.

Figures 2E to 2G show modifications to the device of Figures 2A-2D. The shield of Figure 2A to2D is modified so that it functions like a rapid exchange catheter, with a shortened shield 280 over the main shaft 231. The shaft 281 of the shield 280 could be curved so it sits over the main shaft 231 and a proximal portion of the shield shaft 281 can be used to advance or retract the shield 280. The advantage of the device of this embodiment over that of the previous embodiment is that the user has a greater degree of movement of the shield 280, while maintaining the overall length of the device at a reasonable length. Also, the user will retain control over the needle 201 as the proximal indicator 241 is not covered by the shield.

A potential drawback of the device described in Figures 2A-2G is the potential for the shield 261 to interfere with the freedom of movement of the device within a defect or a trocar, as the shield is on the same side of the device as the needle 201 or is orientated in the same direction as the needle.

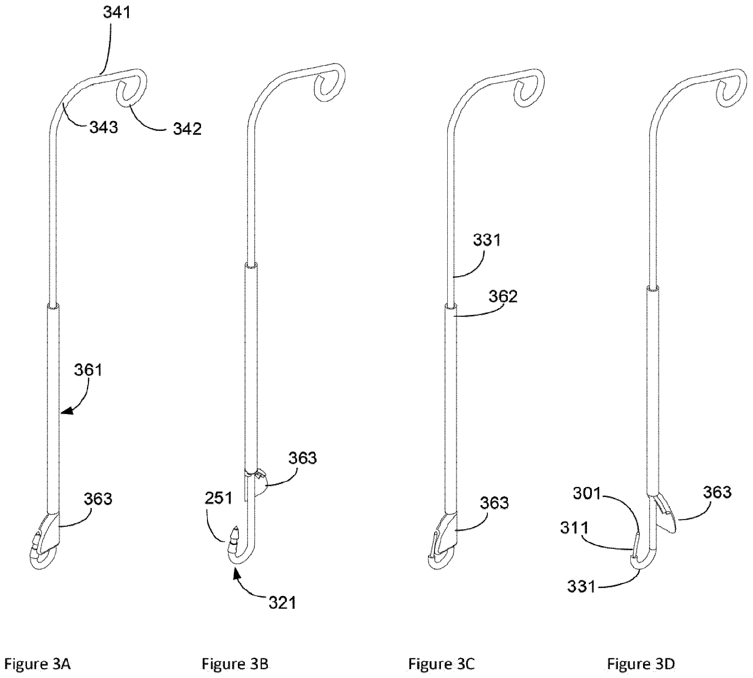

Further examples of a device configured for delivering anchors and suture within the context of the present teaching is illustrated in Figs 3A to 3D. Figures 3A and 3B show the device with an anchor 251 loaded on the device. The anchor shield 361 is in place or extended in Fig 3A and retracted in Fig 3B. Figure 3C and 3D show the device without an anchor, the anchor shield is in place in Fig 3C and retracted in Fig 3D. The shield 361 comprises a proximal shield portion 362 and a distal shield portion 363. In this embodiment the anchor/needle is exposed by rotating the distal shield portion 363 of the shield 361. The distal shield portion 363 rotates to expose the anchor /needle. In this way exposure of the anchor/needle is effected by a relative rotation of the shield and the anchor/ needle between a housed configuration wherein the anchor/ needle are covered or housed by the shield and an active configuration which is achieved through the relative rotation. The path travelled in this relative movement is an arcuate path that in a plane that is perpendicular to the longitudinal axis of the shield. After rotation, as the shield is not on the same side of the device as the anchor /needle the device can be moved more freely to the wound edge. In a modification to this embodiment the shaft 331 of the device could feature a helical thread over which the shield passes, which could move the shield proximally and distally as it rotates.

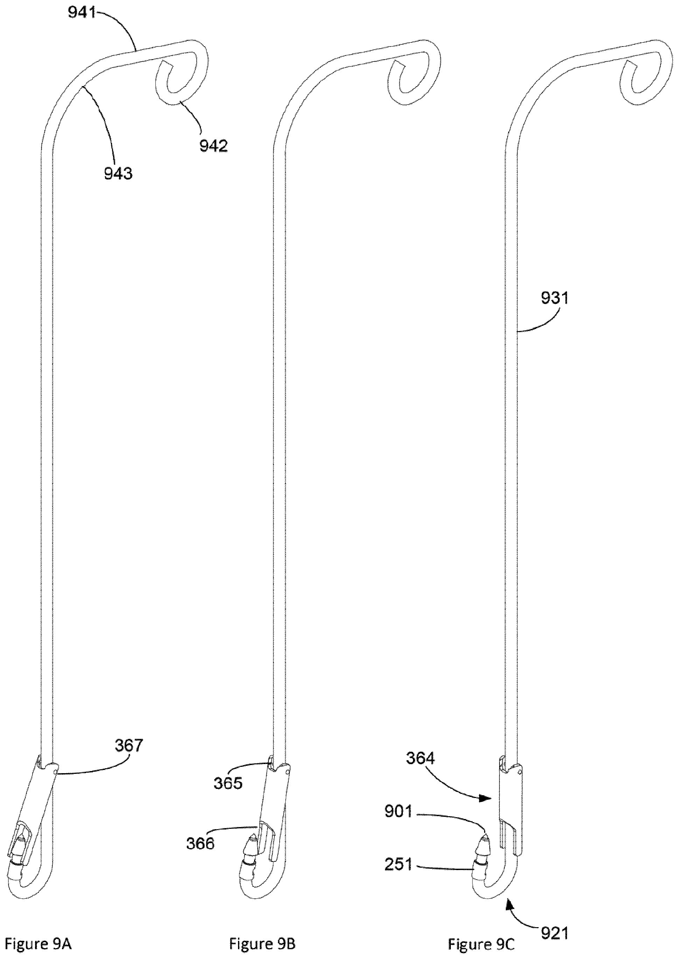

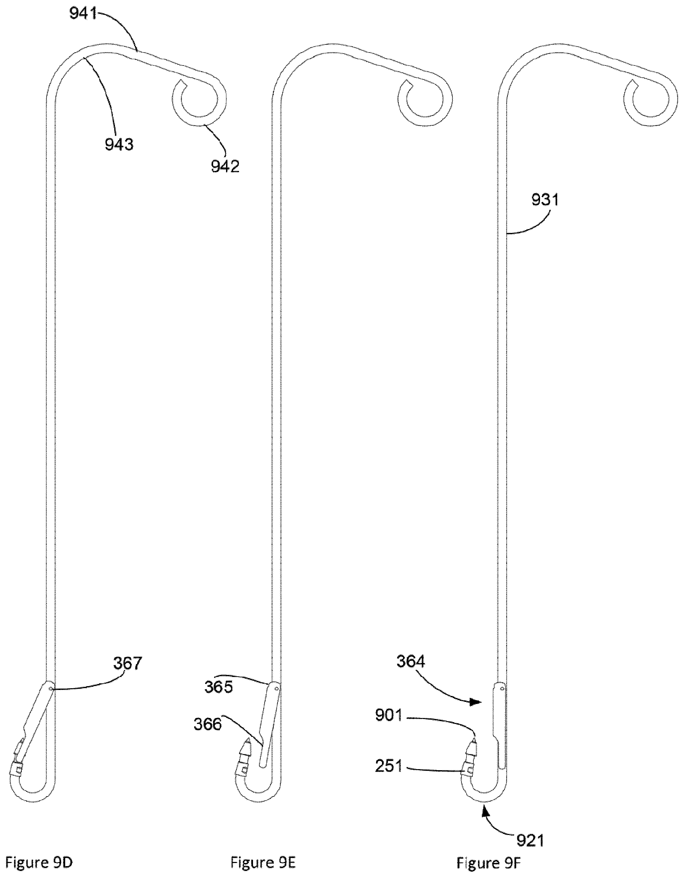

In a modification of the device of Figure 3, the needle tip could be shielded by a carabineer style clip 364, as illustrated in Figures 9A to 9F. The clip would be sprung to protect the needle in its resting configuration. The spring could be overcome by pushing the device sideways against a defect to expose the needle. The clip pivots around a pivot 367, and has a proximal cut-out 365 to prevent interference with the shaft 931 and a distal cut-out 366 to receive the anchor 251. Alternatively a toggle arrangement could be employed to allow the shield to be operated proximally via a pull or push wire. In a further development of this idea a push/pull tube, with an internal diameter greater than the collapsed configuration of the clip could be passed over the clip from the proximal end to collapse the clip and expose the needle tip/anchor tip 901, or be retraced towards the proximal end allowing the clip to close and protect the needle tip/anchor tip again, for removal of the device.

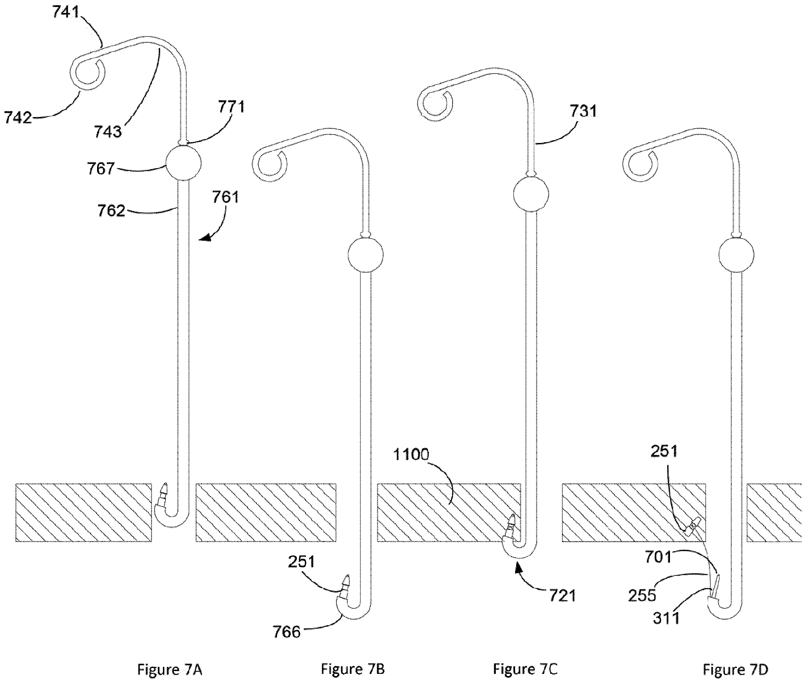

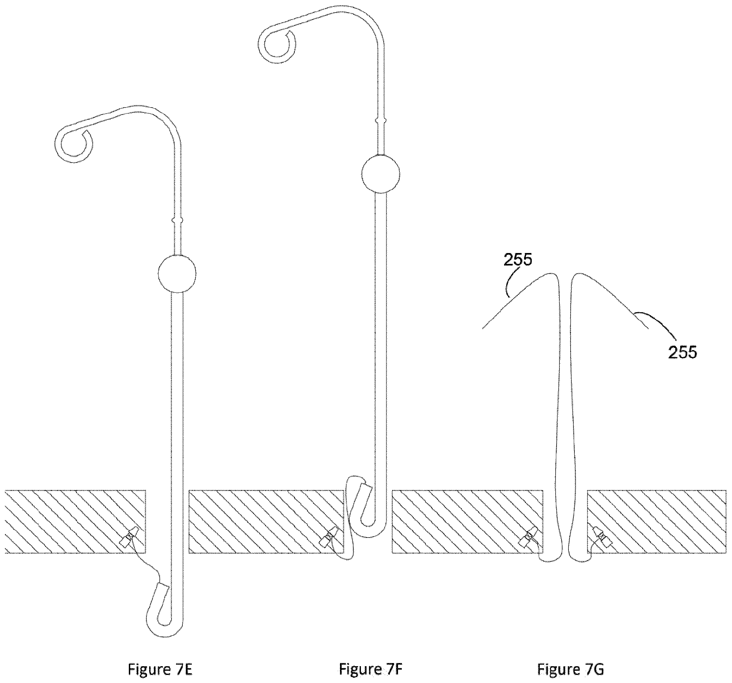

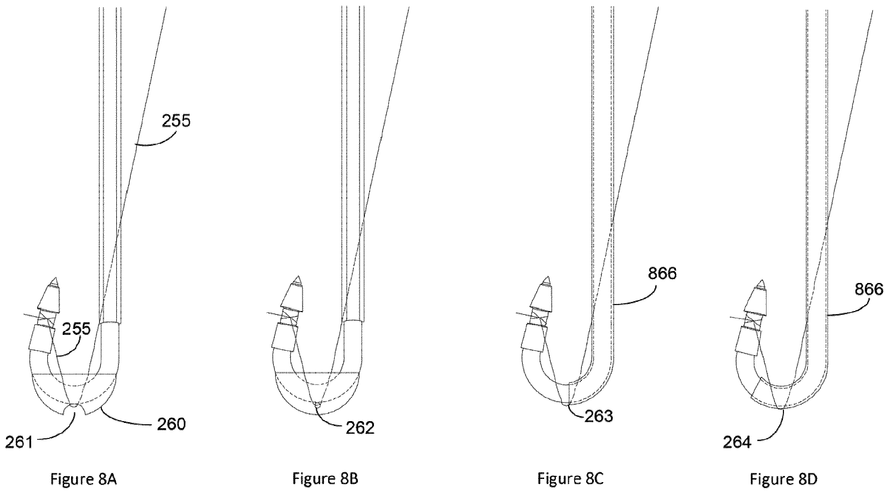

One of the challenges of such a design as described heretofore is that the preloaded anchor 251 sits on the anchor receiving area and could be knocked off if the suture is accidently tensioned, once the shield is removed. A solution to this problem is to tension the suture by pulling the anchor towards the step transition between the anchor receiving portion 211 and the main shaft 231. This could be achieved by creating a notch or eyelet feature at the bottom of the "U" through which the suture passes, and is then retained as a cinch feature at the proximal end of the device. An example of the notch type feature described is illustrated in Figure 8A where a moulded portion 260 is over-moulded onto the wire. A notch feature 261 can be used to maintain tension in the suture while the device is being passed through a trocar or port. The suture is passed through the notch and can be held proximally by the user, or by a proximal suture cinch feature. Alternately this suture could be passed through an eyelet 262 as illustrated in Figure 8B. The features illustrated in figure 8A and 8B could be applied to the device of Figure 7, where a notch or hole is applied to a polymeric tube. This would be particularly useful in a scenario where the device was reloadable, as the device of Figure 7 is designed as a single use device. It would be impractical to thread the suture through the lumen of a polymeric tube during surgery, however running a suture through a notch would be more practical and allow the device to be reused within a given procedure. In a configuration, illustrated in Figure 8C where a polymeric tube 866 is being used to shield the needle post deployment of the anchor, an end 263 of the polymeric tube 263 could be used as a feature to prevent the suture slipping off the u-bend. Alternatively in a further development of this configuration illustrated in Figure 8D, a low durometer polymer could be place in the u-bend area 264. As the low durometer material would tend to be tacky, it would hold the suture in position by friction. A stiffer tube could also be placed at this location and could be configured to enable suture to be threaded though it acting as an anchor for the suture.

Another way to achieve the same result would be to place a tube on the main shaft 231 and through which the suture could run in the space between the outer diameter of the shaft 231 and the inner diameter of the tube. Such a method will retain positioning of the anchor 251 once the suture enters the tube at a point which is more distal than a transition between the anchor receiving portion 211 and the shaft 231.

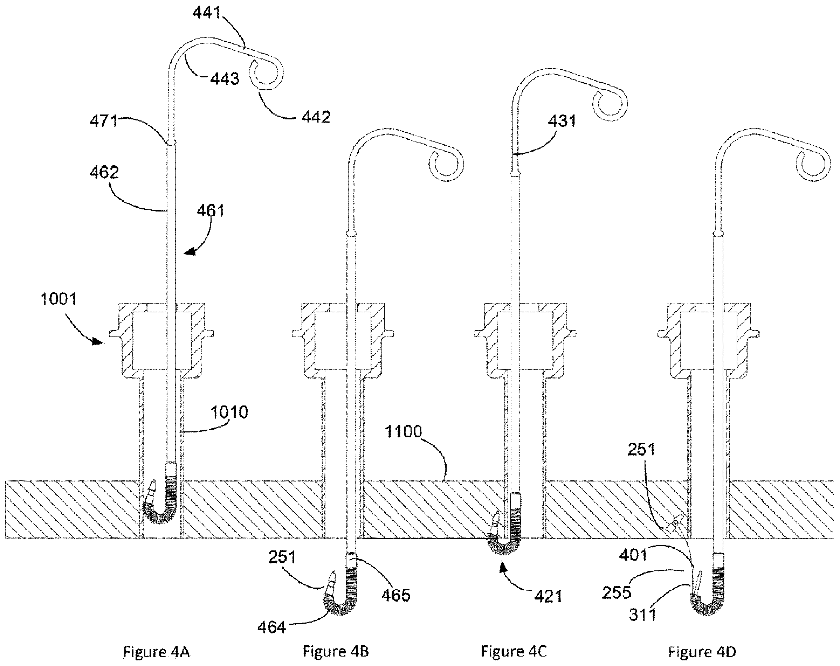

Figures 4A to 4G show additional features that may be incorporated or provided within the context of the present teaching. The device illustrated in Figure 4A is similar to that illustrated in Figure 2 in that it features a needle portion 401, an anchor receiving portion 311, a u-bend portion 421, a shaft portion 431 and a proximal indicator 441 to enable the user to envisage the orientation of the needle. The device also features a shield 461 which comprises a proximal shaft portion 462 and a distal coil portion 464. The proximal shaft portion 462 is hollow and slides along or around shaft 431. Furthermore a crimped portion 471 of the shaft 431 is provided to prevent the proximal shaft portion 462 from moving proximately past a certain point. As the suture 255 is inside the coil 464 and the proximal shaft portion 462, there is no need to shield the needle/anchor for insertion as tension on the suture prevents the anchor 251 from falling off the needle.

Figure 4A shows the device in a Trocar 1001. A trocar shaft 1010 is aligned with the inner surface of the abdominal wall 1100. Figure 4B shows the device passed out of the end of the trocar 1001. The user would then angle the device and pull up to deliver the anchor 251 into the abdominal wall 1100 as shown in Figure 4C. When the user pushes downward on the device, the anchor 251 is left in the abdominal wall 1100.

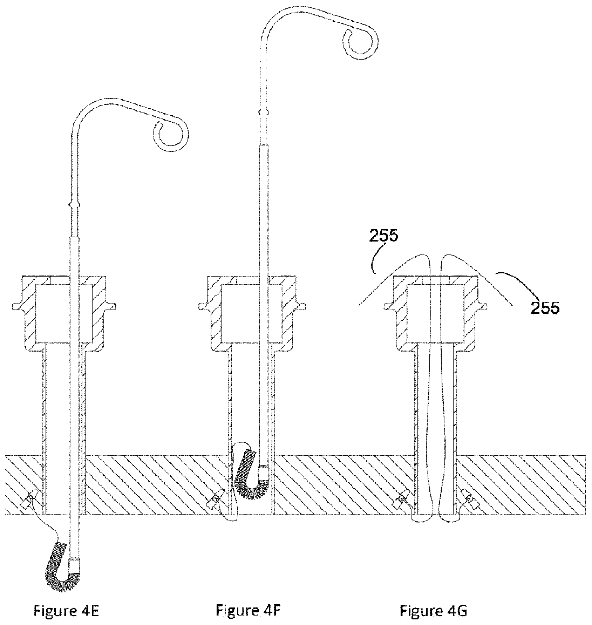

The user can then push down (towards the proximal end of the device) on the proximal shaft portion 462 to advance the coil 464 over the anchor receiving portion 311 and needle 401 as illustrated in Figure 4E. It will be appreciated that the coil 464 functions in a similar way to the shield heretofore described in that it is moveable relative to the anchor/needle to provide selective exposure of the needle. Specifically, it can be seen in Figure 4E that the proximal shaft portion 462 has moved away from crimped portion 471. A visual indicator band (not shown) could be placed on the shaft 431 below the crimped portion 471, which becomes visible as the proximal shaft portion 462 is advanced or moved distally, indicating that the tip is covered. The suture 255 is trailing and is released as the device is removed from the trocar as illustrated in Figures 4F and 4G. The trocar would then be removed and the suture tensioned and knotted to approximate the defect as illustrated in Figure 1B. While it is not shown, the suture could exit the proximal shaft portion 431 such that the suture is visible to the user. Although the device of Figure 4 is shown using two anchors 251, any number of anchors could be embedded in the abdominal wall 1100. For example, larger defects would require more than two anchors. The following is a general procedure for deployment of two anchors using the device of Figures 4A to 4G.
a. The trocar is aligned with the inner abdominal wall surface;
b. The device is passed through the trocar until the u-bend portion is in the inner abdominal space;
c. Angle the device to maximise the fascial recruitment;
d. Once satisfied with the needle position pull up on the device to deploy the anchor
e. Push down on the device to disengage the anchor
f. Advance the shield such that the coil advances over the needle tip
g. Remove the device form the trocar
h. Repeat steps a to g to deploy a second anchor at 180° to the first
i. Remove the trocar.
j. The free ends of the suture, which are outside the body, can be knotted and the knot can be pushed down until adequate approximation is achieved.

The anchor receiving portion engages with an internal lumen of an anchor. The anchor receiving portion could be tapered, and could be used as a method of retaining the anchor whereby the anchor lumen diameter would be smaller the maximum taper diameter and the anchor would be retained by an interference fit. Alternatively the anchor lumen could be also tapered and be used with a tapered anchor receiving portion, or a parallel anchor receiving portion to give a more secure or looser fit as desired.

The proximal shaft portion 462 could be manufactured from a hypo tube or polymer shaft with the coil portion 464 bonded in place with adhesive. Alternately a heat shrink material could be used to form the proximal shaft and be shrunk onto the proximal end of the coil 464. A coil shaft transition 465 between the coil 464 and the proximal shaft 462 could be used to limit distal movement of the coil as illustrated in Figure 4B. This coil shaft transition 465 may be a short section of hardened material that will not deform to advance around the "U" in the u-bend portion 421. Therefore once the coil shaft transition 465 is advanced distally and reaches the bend in the u-bend portion, any further distal advancement is prevented. A short section of heat shrink could be added to the distal tip of the coil 464 to minimise deflection when it is pulled back through a trocar seal. To further simplify construction and minimise cost, the entire shield 766 could be a polymer tube which has enough push ability to advance around the bend without kinking as illustrated in Figures 7A to 7G. Such a result could be achieved with a number of materials like Acrylated Olefin, Polyolefin heat shrink or Tecoflex. Such materials are not intended to be limiting as diameter and wall thickness constraints of the tube and the tightness of the u-bend will contribute to the final material choice. Additionally the tip of the tube could be cut at an angle to minimize the chance of catching on a tight trocar seal, an option which does not exist with the coil option. A multi lumen tube could be usefully employed to reduce the overall profile of the device. The larger main lumen could be used to mover over the shaft, and the smaller lumen could be used to retail the suture.

It will be appreciated that the device of Figure 7 is similar to that described in Figure 4, except that a polymer tube 766 is used to shield the needle tip 701. The device of Figure 7 also includes a proximal indication 741 but in the example of Figure 7a, this proximal indication 741 is shown at a position about 180° to the position of the comparable one in Figure 4 i.e., in the same plane as the indicator 741 but rotated about 180°. It will be appreciated that movement of the indication 741 may assist in visualisation as it may be more intuitive to visualise which side the needle is on relative to the proximal indicator. In addition to the device of Figure 4 with the device of Figure 7, an increased diameter portion is provided in the form of a sphere 767 and is affixed to the proximal end of the shield 762. This sphere 767 functions to give the operator grip when advancing the shield forward or distally. It will be appreciated by those of skill in the art that there are many way of achieving similar results and the one illustrated here is not intended to be limiting. For example, the proximal end of the tube could be heat formed to create a bump feature, or in the case where a hypo-tube was being used a depression could be formed to accept a thumb.

The following gives an example of operable use of a device per the teaching of Figure 7 requiring the deployment of two anchors. It will be understood that larger defects may require more than 2 anchors which will require additional steps:
a. The device is passed through the defect until the u-bend portion is in the inner abdominal space;
b. Angle the device to maximise the fascial recruitment;
c. Once satisfied with the needle position pull up on the device to deploy the anchor;
d. Push down on the device to disengage the anchor;
e. Advance the shield over the needle tip
f. Remove the device form defect
g. Repeat steps a to g to deploy a second anchor at 180° to the first
h. The free ends of the suture, which are outside the body, can be knotted and the knot can be pushed down until adequate approximation is achieved.

In another embodiment, not shown in the Figures, the anchor 251 could be covered by a coil or tube. The diameter of this tube would need to be greater than the combined outer diameter of the anchor and the knotted suture. As with previously described arrangements, the suture could be returned to the proximal end through the internal space between the tube/coil and the outer diameter of the shaft. This approach has the added advantage of being able to protect the exposed needle when passing back through the trocar after deployment of the anchor. This also allows the device to be packaged without a tray in a pouch. In the previous embodiments, a tray needs to be used to protect an exposed needle when the device is packaged in a pouch - the device is packaged without an anchor on the needle. In this embodiment, as the exposed needle is sheathed by the coil or tube, no such tray is needed. The procedure for using this device is as follows:
a. The trocar is aligned with the inner abdominal wall surface;
b. The device is passed through the trocar until the u-bend portion is in the inner abdominal space;
c. Retract the coil/tube to expose the anchor/needle tip
d. Angle the device to maximise the fascial recruitment;
e. Once satisfied with the needle position pull up on the device to deploy the anchor
f. Push down on the device to disengage the anchor
g. Advance the coil/tube over the needle tip
h. Remove the device form the trocar
i. Repeat steps a to g to deploy a second anchor at 180° to the first
j. Remove the trocar.
k. The free ends of the suture, which are outside the body, can be knotted and the knot can be pushed down until adequate approximation is achieved.

Another exemplary arrangement that can be usefully employed within the context of the present teaching and is similar to that previously described except the coil/ tube would not cover the anchor could incorporate a tearaway cover. This could be used to protect the tip if there was a strong desire to pack the device without a tray. This cover could be a small moulding which is suitably coloured (e.g., red) to make it obvious it must be removed before use. The anchor would be maintained in position by tension on the suture, being passed through the inner diameter of the coil/tube. The device would be passed through the trocar seal and used to deploy the anchor in the desired location. After use the spring /tube could be advanced to cover the needle, ensuring that the needle does not get caught in the trocar seal or port during removal of the device, and that there is no exposed sharp end.

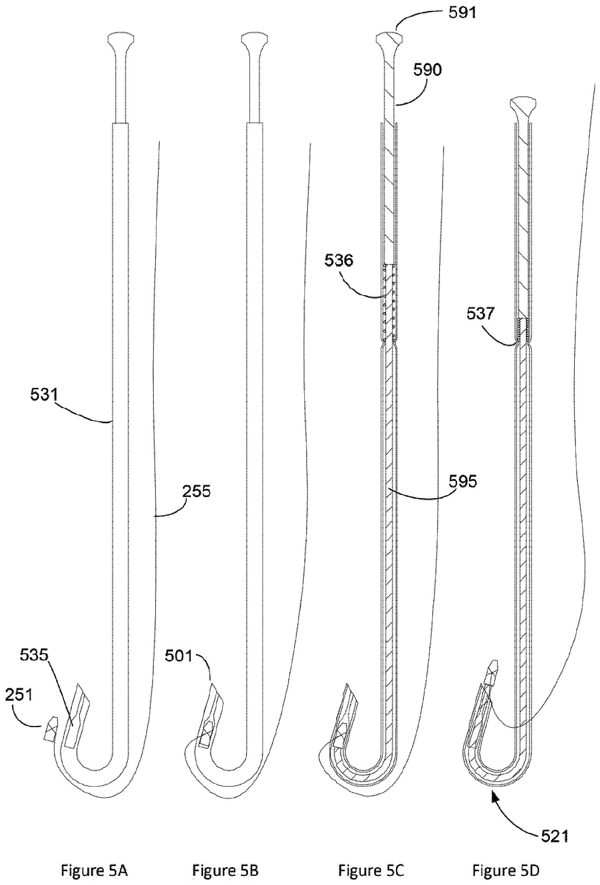

Figures 5A-5D illustrates another arrangement of a device provided in accordance with the present teaching. In this example, the device is provided as a reloadable device. The device comprises a hollow shaft 531 with a u-bend portion 521 and a needle portion 501. While the needle is illustrated in this example away from the shaft it will be appreciated that having the needle oriented 180° to this location would have an advantage when the device is being removed, as it would be less likely to catch. The device has a side window 535 at the end of the u-bend portion, through which an anchor 251 can be side loaded. To minimise the chances of injury the side window should be kept as far away from the tip as possible. Inside the hollow shaft 531 there is a pusher comprised of a flexible element 595 and a rigid component 590. A compression coil 536 interacts with a crimped portion 537 of the hollow shaft 531 to limit movement of the pusher when a proximal handle 591 is pushed relative to the hollow shaft 531. Upon release of pressure on the handle 591, the spring returns the pusher to the default position. Once loaded the device can be passed through a trocar. When the user deploys the anchor 251 by pressing the handle 591 the anchor is delivered into the abdominal wall. On releasing the handle 591 the pusher retracts, leaving the anchor behind. The device is then removed from the trocar and reloaded for the next anchor. The flexible portion 595 of the pusher could be manufactured from a polymeric tube, rod or coil. If construction of the pusher was robust enough the pusher could be left extended to facilitate remover through a trocar.

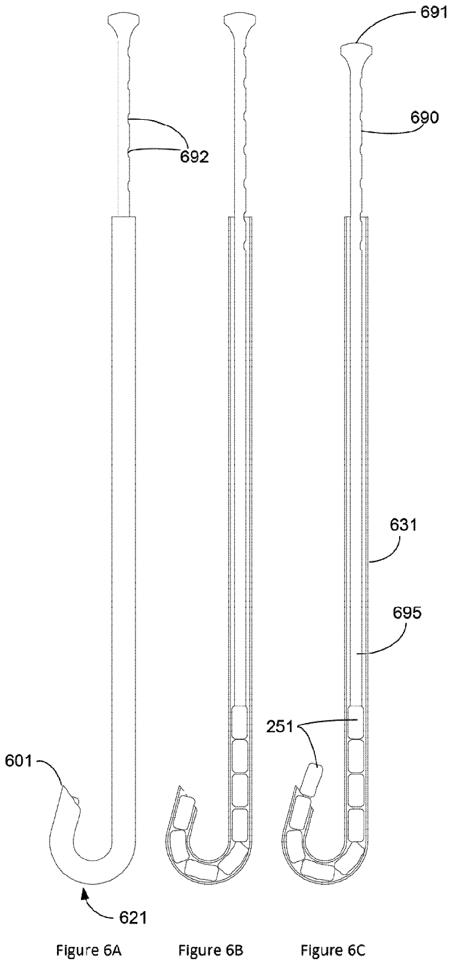

Further examples of devices provided in accordance with the present teaching are illustrated in Figures 6A to 6C where multiple anchors 251 are preloaded. The device comprises a hollow shaft 631 with a u-bend portion 621 and a needle portion 601. A pusher portion comprises a shaft portion 695 and a proximal portion 690 which features a push handle 691 and indentations 692. Multiple anchors 251 are illustrated preloaded on the device. The proximal portion 690 begins in a retracted position and is advanced, relative to the hollow shaft 631 as each anchor is deployed. The shaft portion 695 may be flexible to enable it to track round the u-bend portion 621. Each anchor 251 will have a suture attached (not shown). This suture may be run back though the inner lumen of the shaft 631 exiting at the proximal end of the shaft 631. The indentations 692 on the proximal end of the pusher shaft 695 could be employed to provide tactile feedback to enable the user to identify when an anchor is deployed. This device has the advantage over the devices of the previous embodiment described with reference to figure 5, which is side loadable in that it does not need to be removed from the trocar or wound site after each anchor 251 deployment. A user skilled in the art will appreciate that any of the shielding methods described earlier could be combined with the devices of Figure 5 and Figure 6.

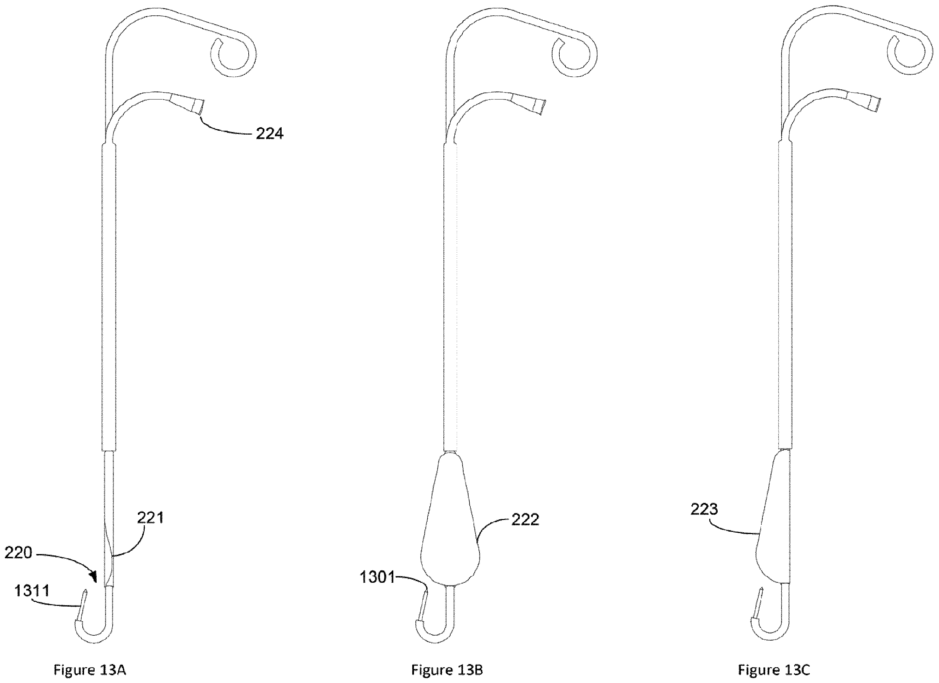

A further embodiment, illustrated as Figure 13A to 13B involves having a balloon 221 on the shaft proximal to the needle tip 1301, and that when fully inflated 222 it does not contact the needle, but creates a transition over which a trocar seal has to stretch as the balloon is pulled though, which prevents the seal catching on the needle tip. Alternately the balloon could be eccentric, as shown in Figure 13C such that the inflated portion 223 is in the area above the needle 220 (Figure 13A).

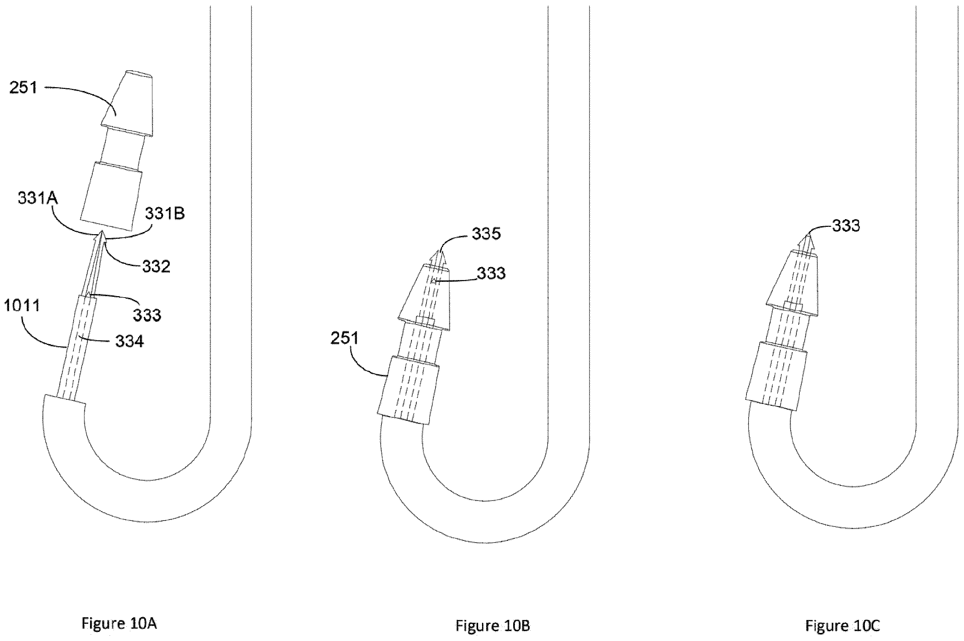

In an embodiment where the device is reloadable, a needle engaging portion 1011 could be made lock the anchor onto the needle as illustrated in Figure 10A to 10C. In such a configuration there is a split needle portion comprising of a first side 331A and second side 331B. A wire 334 has a needle tip 333 and can be pushed though the split portion to expand the barbs 332 such that the diameter of the expanded barb is greater than the anchor lumen diameter. This holds the anchor in place and there is no need for running the suture through a tube as shown in Figures 4 or 7, or for any of the suture retention features of Figure 8 as the anchor is secured on the device. When the wire is advanced as in Figure 10B a space 335 is created between the split portions, until the wire is advanced to its final position, where the tip 333 fills the space 335. In an alternate arrangement a similar concept could be used where a wire could passed through the anchor lumen, and in doing so expand a split portion to create an interference fit on the inside lumen of the anchor without relying on barbs to maintain the anchor position. A further embodiment would be to redesign the anchor portion to have an expandable portion, such that when a compressive load is applied, this portion expands to relieve the interference fit and allow the anchor to be deployed in tissue.

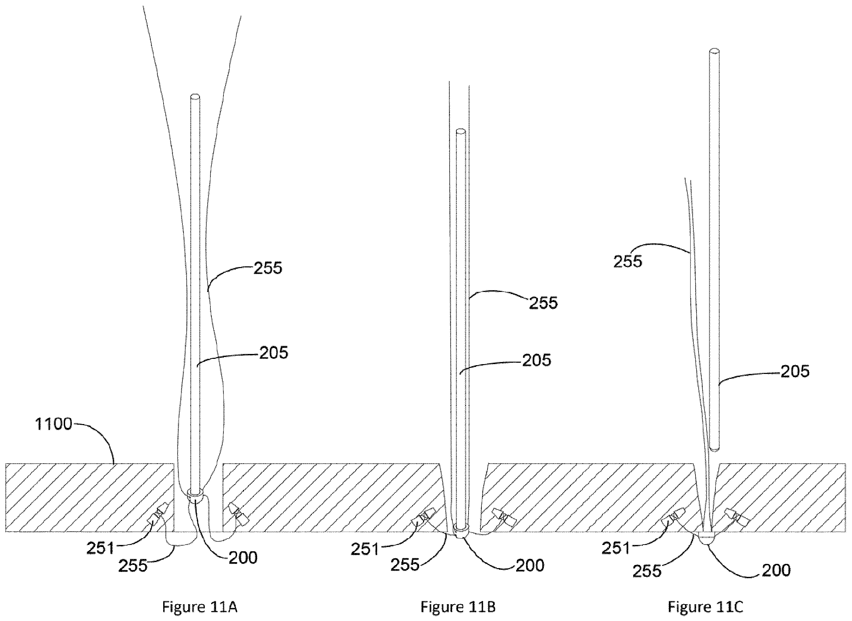

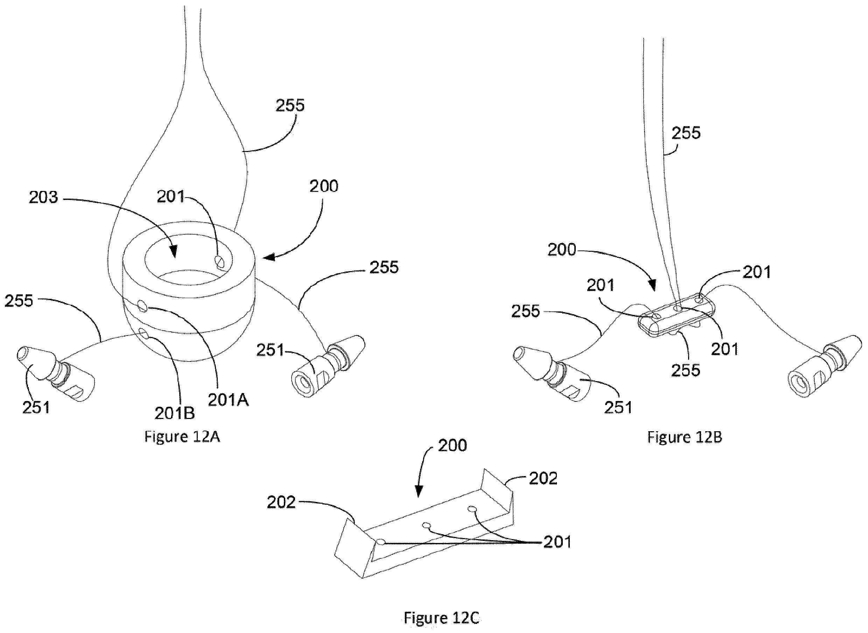

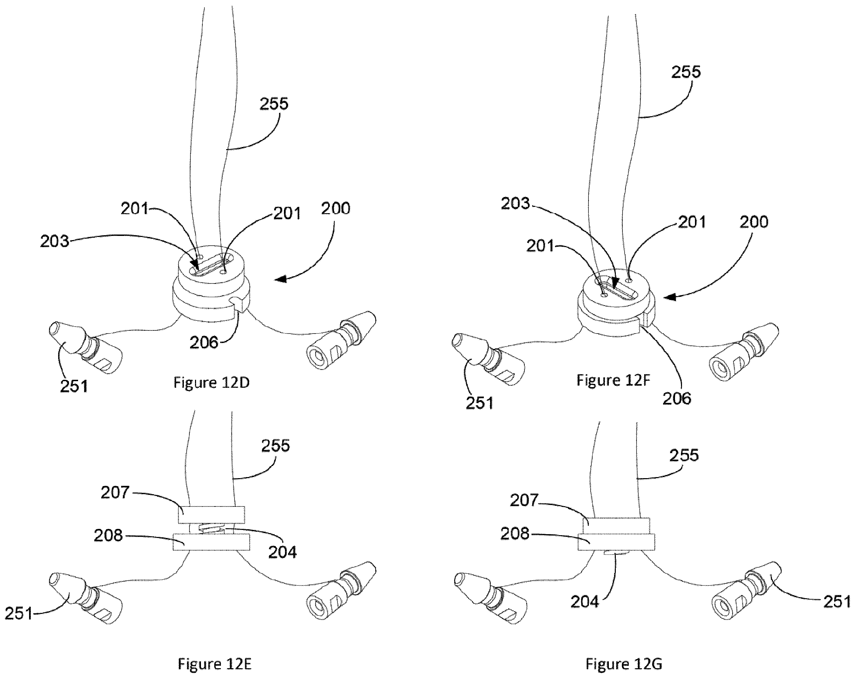

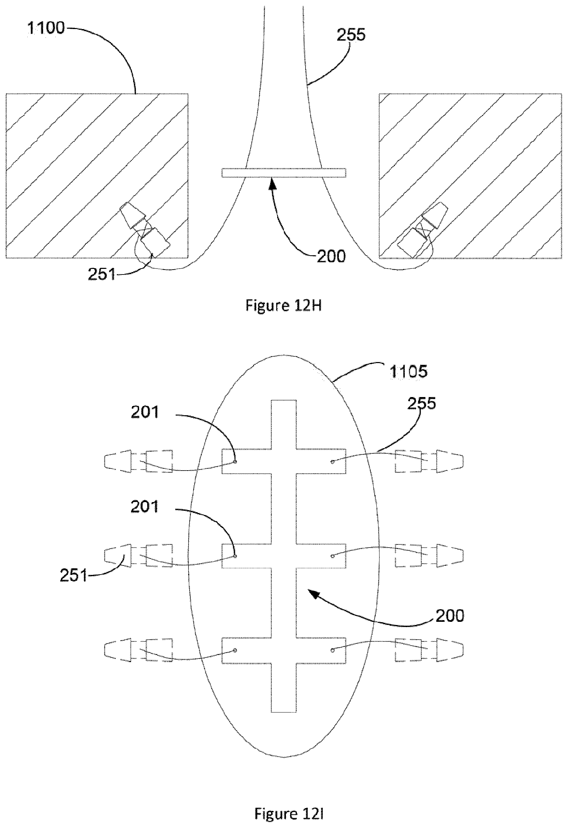

In closing a defect where a pair of anchors are deployed it would be advantageous to have a feature to maintain tension on the anchor. Such a feature is illustrated in Figure 11C where suture 255 is passed through a pair of holes on a bioabsorbable cinch 200. The cinch could be configured to have a geometry designed to plug a defect as well as maintaining tension on the suture. Figure 12C illustrates a further suture cinch which is co-operably used with a driver rod 205 (Figure 11A). The cinch features a pair of holes 201A and 201B on one side for one suture. A similar pair of holes are disposed on the opposite side for the second suture. The cinch also has an engaging portion 203 recess, which mates with the driver rod. Variations on the geometry are illustrated in Figures 12B and 12C. Figure 12C also illustrates a cutting edge 202 which assists in embedding the device in the fascia, once the suture is tensioned. While illustrated as an edge, this feature could also take the form of needles, or barbs. A further embodiment is illustrated in Figure 12D and E in an open configuration and Figures 12F and 12G in a closed configuration. The top half 207 and bottom half are attached via the threaded portion 204. The thread could be on either portion. Rotation of the top half, relative to the bottom half locks the suture, by creating misalignment of the suture channels 201. A driver rod would cooperate with the cinch seat in the recess 201. To ensure both that one half can be held, while the other rotates a notch 206 could engage with the non rotatable portion of the driver rod. Such a cinch could also be usefully employed to close longer defects as illustrated in Figure 12H and Figure 12I. This geometry would minimise the risk of tissue ingress into the defect, as the device would create a physical barrier. The device could be used with several pairs of anchors as shown, or just a pair at either end.

Figure 11A to Figure 11C show a cinch in use, where the rod 205 is used to push the cinch 200 deep into the defect. The exposed ends of the suture are tensioned as the rod is advanced, until the point where the cinch is at the bottom of the defect as illustrated in Figure 11C. Such a mechanism could be used to close a defect as the cinch and anchor/suture are bioabsorbable. Additionally such an arrangement could be used to maintain initial tension in a deep defect, while the surgeon uses the suture to throw additional knots down to the cinch.

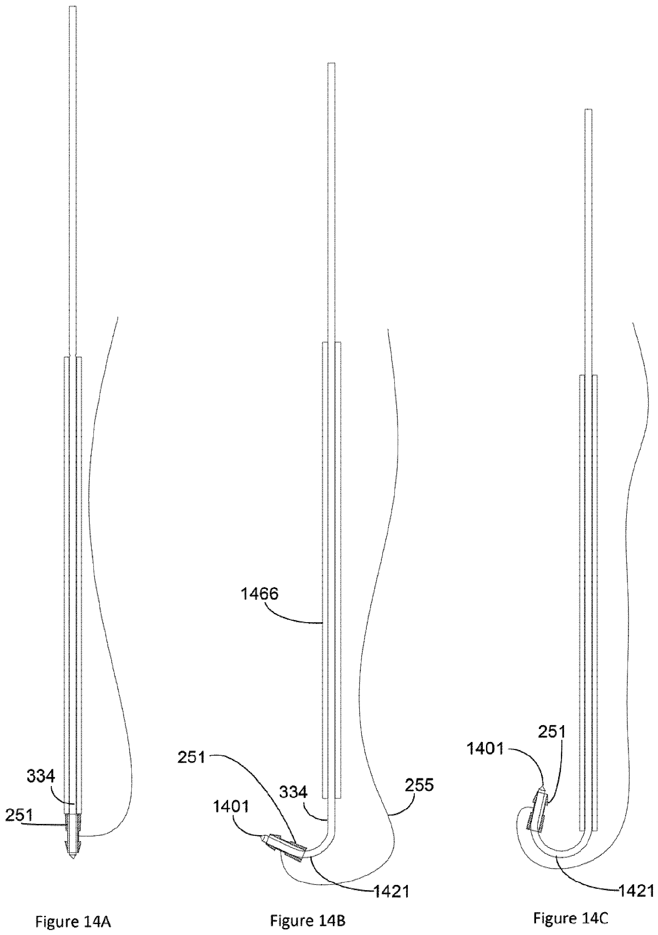

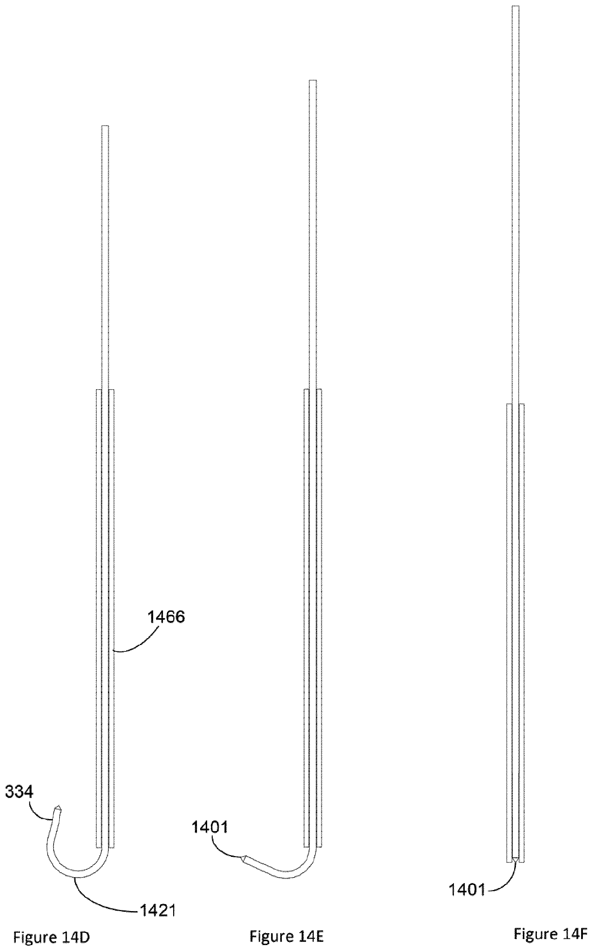

The device on Figure 14 features a Nitinol wire which is pre formed in a u-shape. It will be appreciated that Nitinol is an alloy of nickel and titanium that exhibits properties of a shape memory material including the capacity to deform and return to a predetermined shape on application and removal of a suitable force. In this context any shape memory material that exhibits these characteristics may be considered suitable. A hypotube 1466 is used to maintain the wire in a straight configuration for deliver, and an anchor 251 is loaded onto the distal end of the wire. When the hypotube is retracted the Nitinol wire adopts the u-bend configuration 1421. The anchor does not pass around the u-bend due to the curvature of the wire relative to its lumen. Once the anchor has been deployed the hypotube 1466 can be advanced to shield the needle for removal. Such a device would have particular advantage in hernia mesh applications to deploy anchors to affix the mesh to the inner abdominal wall. Such a device could adopt reloadable anchors whereby the hypotube is removable from the proximal end. An anchor could be passed over the Nitinol wire from the proximal end. The hypotube could be replaced and be used to push the anchor around the u-bend as the Nitinol wire is retracted into the hypo tube. A stopper could be crimped onto the Nitinol wire and interact with a bump on the proximal end of the hypotube, to restrict forward motion of the hypotube, preventing premature deployment of the anchor.

Figure 15 is a further embodiment of a device, shown pre deployment in Figure 15A and post deployment in Figure 15B. The device shares many features in common with the previously described devices. In this arrangement, the device 1500 comprises a handle portion 1510, a main shaft 1505, a u-bend portion 1530 and a shield 1530. In this aspect of the present teaching the device is configured such that the functionality of the shield can be extended to provide a housing for an anchor 1251. The shield may be provided as a removable shield. In this aspect, it is possible by replacement of a shield at the end of the shaft with one or additional shields which are pre-loaded with new anchors to use the same device with multiple anchors. Alternately the device could be provided with a non replaceable shield, into which the user would place an anchor. The insertion of an anchor into the shield could be done as a manual process or could be facilitated through use of a reloading tool. A simple tubular housing and push pin configuration would be an example of such a tool.

In Figure 15B a top surface 1540 of the shield is labelled. Upon delivery of the device through a port and into the abdominal cavity, this surface can be pulled against the inner abdominal wall surface for example to establish the delivery location and can therefore be considered an abutment surface. Once the desired delivery location is selected, the user can depress a button 1512, 1513 (labelled in Figure 15A as item 1512 in its non-depressed position and in Figure 16A as item 1513 in its depressed position) which will be located externally of the abdominal cavity during use. The button can be coupled to a driver portion of the device and can be used to facilitate an unlocking of the driver portion of the device from the shield 1520 so as to allow relative movement of the two. In this way and as seen in Figures 16A, 16B, with the application of upward pressure on the handle 1510, the user can expose the anchor, which is driven a fixed distance into the abdominal wall. The device could be configured such that continuous application of force onto the button 1512 is required to facilitate the presentation of the anchor into the abdominal wall. In an alternative embodiment the needle could be spring so that a certain force threshold would have to be exceeded to enable deployment. This would prevent inadvertent deployment of the anchor during delivery.

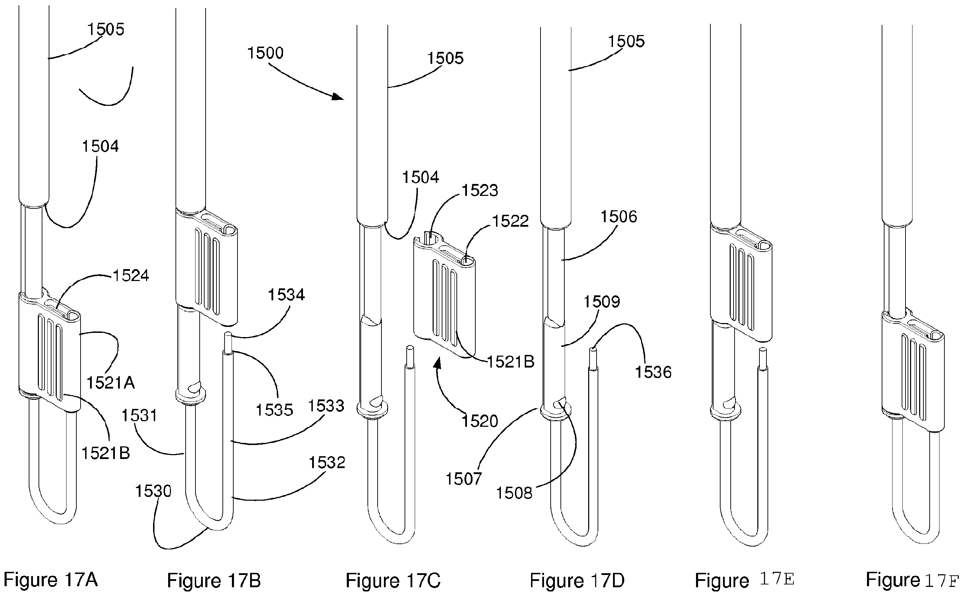

Turning now to Figures 17A to 17E. This series of images illustrate an exemplary process flow whereby a shield is removed and replaced with another preloaded shield. Figure 17A shows the shield in place on the device. To remove the shield the user would grip the shield by the textured surfaces 1521 A and 1521B and slide the shield up until it abuts with a step on the shaft 1504, as illustrated in Figure 17B. Also visible in Figure 17B is a driving wire 1531 which has a u-bend form 1530 between two parallel wire sections 1531, 1532. The wire portion 1531 slides inside the shaft 1505. The portion 1532 slides within the shield. A turned down portion 1534 is disposed at the end of the wire, and the difference in diameter between this promotion and the portion 1533 creates a seat or step 1535, which engages with the proximal end 1253 of the anchor 1251 (Figure 16B). Previously disclosed embodiments (the device of Figure 2 for example) featured a sharp tip on the wire, which acts as the cutting edge for delivery of the anchor into the abdominal wall. In the anchor of this embodiment the anchor tip 1252 is the cutting edge and the wire does not extend fully through the anchor body. In this scenario, a further embodiment is envisioned where the wire is not stepped, but is a constant diameter similar to that of section 1534. In this instance the wire would bottom out on the lumen at the proximal end of the anchor. The advantage of such a construction would be a reduced cost wire due to less material and absence of a turn down operation. However this may impact on the robustness of the device, and for a device where multiple uses are envisioned the additional cost of the turn down portion may be negligible, Additionally the end of the wire 1536 (Figure 17D) is illustrated with a square end, but this is not limiting as a conical or ball nose finish may be better adapted to aligning the wire with an anchor when the shield is replaced.

In Figure 17D it can be seen that towards the distal end of the shaft 1505, there are two distinct profiled areas on the shaft, i.e. 1506 and 1509. The shield is slideable along each of these profiled areas and the movement is limited on the top of the profiled areas by a step 1504 and at the bottom of the profiled areas by a step 1507. The length of area 1506 is comparable to that of the shield and the shield can be removed sideways as shown in Figure 17C.

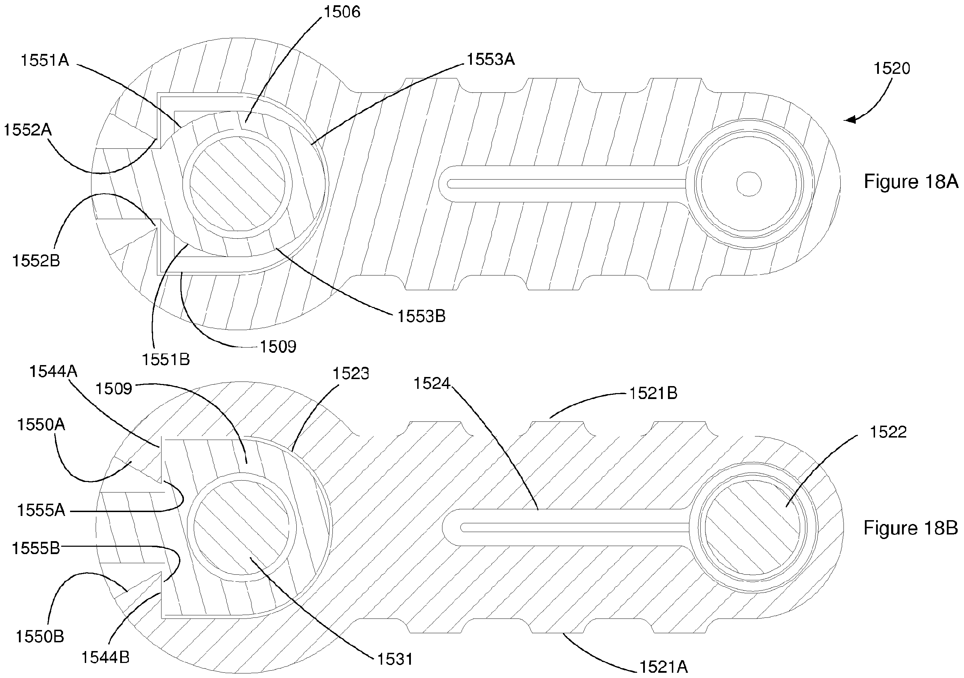

Turning now to Figure 18, which is a section view of the device with the shield aligned with portion 1506, it can be seen that the geometry of the shaft in this area 1506 is configured to provide an elliptical profile. The surfaces 1551A and 1551B which are presentation surfaces facilitate removal of the clip by providing a smooth surface over which the inner protrusions 1552A and 1552B of the shield can pass. Once removed the spent shield can be disposed of or reloaded and then re-presented at a later time. Another preloaded shield can be removed from the packaging and be aligned with the area 1506 (Figure 17). The shield is provided with presentation surfaces 1550A and 1550B which are configured to be extendible between a normal unexpanded configuration to a larger expanded configuration which is adopted during the locating of the shield onto the device and during the removal of the shield from the device. The shield is at least partially flexible in this area to allow progression from the unexpanded to expanded configurations. In the unexpanded configuration the surfaces 1550A, 1550B are arranged relative to one another so as to correspond with the maximum diameter (on the minor axis of the elliptical profile) of the shaft 1506. As the shield is pushed sideways onto the shaft 1506, the surfaces 1550A, 1150B move apart from one and other and surfaces 1553A and 1553B facilitate smooth movement of the inner protrusions 1552A and 1552B over the shaft.

After locating the shield onto the driver tool, the user would, while gripping the shield by textured surfaces 1521 A, 1521B slide the shield down until it abuts with a distal step on the shaft 1507. The shield features inner surfaces 1554A and 1554B as illustrated in Figure 18B, and these surfaces mate with the surfaces 1555A and 1555B of the portion 1509. These mating surfaces, coupled with a shaft profile which is matched to diameter of the inner lumen of the shield 4523 ensure no rotation of the shield is possible once located on this portion of the driver. The shield when advanced to the distal stop 1507, clicks in place as a protrusion 1508 on the shaft 1509 interacts with a depression on the inner surface of the shield. It will be understood that the male and female surfaces described on each of the shield and driver tool could be reversed and still facilitate the same engagement between the shield and device.

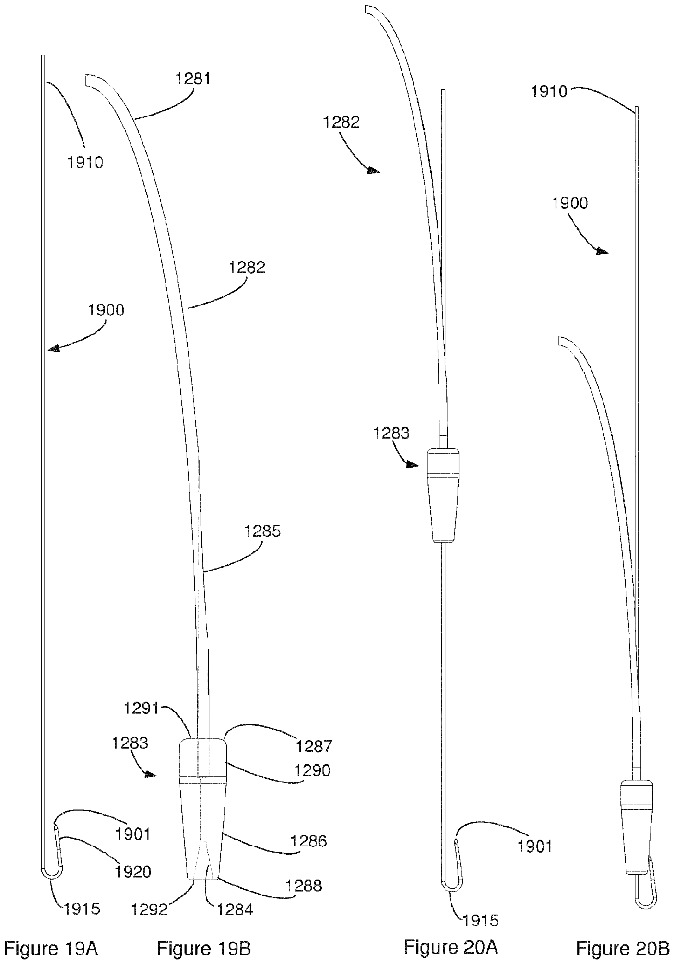

Figure 19 shows a further embodiment of a device similar to that described earlier as Figure 2 which may be usefully employed in retrieving a driver device after deployment of an anchor. The device in this arrangement comprises a retrieval device 1282 which comprises a handle portion 1281, a distal portion 1283 and a Rapid Exchange (RX) portion 1285. The distal portion features a tapered surface 1286, which facilitates expanding the seal of a trocar or the tissue in a port, to allow the largest profile section 1290 to pass through. The proximal 1291 and distal ends 1292 both feature a radius, 1287 and 1288 respectively, which also aids insertion and removal of the device. However, this geometry is not limiting and the geometry could be further improved by adding an extended taper at the proximal end 1291 of the distal portion 1283. Alternately, a spherical form could be used like the example in Figure 21B.

To use the device the user would pass the hook 1900 with a preloaded anchor through the defect or port and pull up the needle 1901 into the abdominal wall at the desired location to deploy the anchor. Then the distal end 1292 of the retrieval tool 1282 is passed over the proximal end of the hook 1910 The distal end of the retrieval 1282 may also include an internal funnel 1284, This aids placing the device over the proximal end of the hook 1910. As the user pushes the retrieval device 1282 further over the wire, the proximal end of the hook 1910 will exit the RX port 1285 of the retrieval tool 1282. Now the user can hold the proximal end of the hook 1910 as the retrieval tool is advanced the rest of the way over the hook. The retrieval tool is advanced until a stop is felt, this occurs when the tapered section 1285 comes in contact with the sharp tip 1901 of the hook 1900. To remove the device from the defect, the user then pulls on the proximal end of the hook and as the device is pulled through a port or defect, the larger diameter potion 1290 of the retrieval tool 1282 provides resistance, against the action of removal and maintains the taper 1286 in contact with the sharp tip 1901. Once removed the retrieval tool 1282 can be removed from the hook 1900, and be used again with additional hooks.

Figure 22 shows another embodiment of a device which has a shield to protect a needle tip, to facilitate safe removal from a port or defect. These images do not show the anchor, as it has been well described previously, but it will be appreciated that an anchor when provided could be mounted on the stepped down portion 1831 of the wire. The device 1800 comprises a shield 1840 which is connected to a hollow shaft 1805 and a handle portion 1810. A wire with a U-bend 1830 terminates with a sharp tip 1832, and on the other side of the u the wire is disposed within the lumen of the shaft 1805. Proximally the wire is connected to the handle portion 1820. The handle portion 1820 is shown in Figure 22A in the non active position and is sprung in this position by a spring in the handle (not shown). In Figure 22B, the handle portion 1820 is shown depressed, this moves the wire distally, and moves the tip 1832 inside the lumen 1841 of the shield 1840. This then facilitates safe removal of the device 1800 from a port or defect as the sharp needle is covered, avoiding the needle tip catching on a trocar seal, or tissue within a defect. The curved surface 1842 further facilitates removal by creating a smooth transition between the shaft and the largest diameter of the shield. This curved surface 1842 also serves as a depth stop as it creates an interface where it meets with the vertical portion of the wire 1833. The button 1820, when released will spring back to its initial position. Alternately a simple catch feature could be employed such that once depressed the catch holds the button, shielding the anchor, until a catch release is depressed. Such a safety feature may be preferred as there is a sharps risk associated with an exposed needle tip, especially after use. The shield also incorporates a hole 1830, through which the trailing suture from the anchor could be passed. Alternately, the curved portion of the shield 1842 could be omitted, which would leave a void in between the lower edge of the anchor shield 1843 and the u-bend of the wire 1830.

This device could be further adapted if the diameter of the shield lumen 1842 was increased to receive an anchor. In such a scenario the shield would actively cover the anchor and needle tip. When the device is inserted it would be configured much like it is in Figure 22B. A catch could be released to release the handle portion 1820, which is sprung, such that it would retract the shield. The device could then be used to deploy the anchor, before the button 1820 is depressed, triggering the catch, shielding the anchor and making it safe for removal. Upon removal, because the shield covers the sharp tip, the user could then take an anchor and push it into the lumen 1840, and thread the suture through the hole 1830, and proceed to re-use the device. An alternative way of securing the anchor within the shield 1840 is illustrated in Figures 25A and 25B. In these configurations the slot 1843 is sized such that the suture is slightly compressed as it is pulled though the slot. A radiused lead in 1844 enables the user to guide the suture in though the slot. When the suture is pulled into the bottom of the slot, it is free to move with the opening 1845, but is prevented from falling out due to the tightness of the slot.

Another device to deploy anchors is depicted in Figure 23 and 24. In this device which shares features with that described previously, first and second anchors can be deployed from the same device- either concurrently or sequentially at two different locations within the abdominal wall. To facilitate the delivery of multiple anchors first 1950A and second 1950B wires are provided, each wire forming a needle with a u-bend. In this arrangement each wire or needle 1950A, 1950B features a sharp tip 1952 and a step down portion 1951, which receives an anchor, with the step 1953 engaging with the anchor to transmit push to the anchor. This is similar to that described before and it will be appreciated that the wires could also be configured to provide a seat for an anchor which then self-pierces the abdominal wall- such as was described above with reference to Figure 17. The wires 1950A, 1950B are housed in a shaft 1935 which provides a shield for the tip 1952. The shaft 1935 has side openings 1941A and 1941B which allow the u bend and needle potion of each wire to rotate from being housed in the shaft as shown in Figure 23A to being deployed as shown in Figure 23D. In this way relative movement- in this configuration rotation-between the needle tip 1952 and the shield defined by the shaft facilitates the movement of the tip from a shielded or housed configuration to a deployed or active configuration. In the deployed configuration the tip is exposed and can penetrate the abdominal wall or other tissue parts. In the shielded configuration the tip is shielded or housed by the shield and as such the risk of inadvertent contact between the tip and the abdominal wall or other tissue parts is minimised.

As shown in Figures 24A and 24B, the shafts 1950A and 1950B terminate in a pair of gear wheels 1960A and 1960B which are connected to the wires. A central gear (not shown is mounted within the handle portion 1940 and is provided such that rotation of the handle 1940 through interaction of the gears effects rotation of the wires. Once deployed the user pulls up on the device to penetrate the abdominal wall with the needle tips 1952A and 1952B, and subsequently the anchors. To remove the device safely from the defect the user rotates the handle in the opposite direction, and the needle tips are rotated into the side openings 1941 of the shaft 1935, this shielding the sharp tip. The advantage of such a device is that it facilitates delivery of two anchors simultaneously to either side of a defect at an angle of 180, ensuring a consistent and repeatable result. It will be appreciated that a simpler construct could also be provided whereby a common delivery shaft which houses two wires on which two anchors are provided, the wires being arranged within the shaft such that the anchors extend from different sides of the shaft. In this way manual retraction of the individual wires could facilitate delivery of multiple anchors.

Figure 26 shows another example of a delivery device that uses a relative rotation of the needle tip and the shield to effect movement of the tip and anchor from a housed or shielded configuration to a deployed or active configuration. In this exemplary arrangement the device is configured to deploy two anchors 251 Each of the anchors are located on a respective drive wire 2075 which are coupled but moveable relative to a shield. In this configuration the shield comprises a main shaft portion 2040 and a shaft cover 2035. Each wire 2075 has a u-bend portion 2050 provided proximal the anchor 251 that is seated on the wire. A second bend 2072, typically of the order of about 90°, is provided at a handle portion 2041 of the wire. In assembly, each wire 2075 is located within a longitudinal channel 2043 defined within the shield main shaft portion 2040 of the shield. The main shaft portion 2040 is then covered with a shaft cover 2035 that is provided in the form of a covering tube wall and which is seated over the main shaft portion. A handle 2060 passes through the shaft cover 2035 and a corresponding aperture 2046 in the main shaft portion 2040 thereby retaining the two in relative alignment and position.

As shown in Figure 27C each wire 2075 features a step-down portion 2051 onto which an anchor may be loaded and seated prior to delivery into the abdominal wall. Per the previous examples of anchor delivery devices, the anchor is coupled to suture and in this configuration the suture may be configured such that a suture tail pass through a central hole 2045 in the main shaft portion to the opposite side of the device and allows suture (not shown) from the anchor assembly 251 to pass though the main shaft portion. Once the central hole 2045 is distal to the point on the anchor at which the suture is secured, the suture will drag and ensure that the anchor is not accidently dislodged from the needle during delivery of the device through the abdominal wall.

The main shaft 2040 may also comprise a cut-through portion 2044 which defines a window portion 2036, in which the u-bend portion of the wire is seated and from which the tip of the wire will rotate when adopting the deployed configuration.

To use the device of Figures 26 and 27 the user passes the tip portion through the abdominal defect as illustrated in Figure 28A. Once the window portions 2036 are inside the interior of the abdominal cavity, the user can rotate the device to adopt a desired orientation. The handles 2060 provide the user with a visible guide to the orientation of the needles when the device is in the open position as they extend out from the main shaft body in the same direction that the needle tips will, once deployed out from their housed or shielded configuration. The deployment levers are formed from the wire 2075, and are show here fitted with a push fit tube, which covers the wire ends. The user, once happy, with the orientation of the device rotates deployment levers 2070A and 2070B until they are aligned with the handle 2060, and in doing so rotates the needles 2052, ready for deployment. To deploy the anchors into the abdominal wall 1100 the user pulls firmly upwards on the handle 2060 until the anchors 251 are deployed within the abdominal wall, as shown in Figure 28C.

The user then pushes the device downwards to retract the needles from the abdominal wall, Figure 28D. To remove the device from the abdomen the user rotates the deployment levers 2070 to their starting position, shielding the needles and allowing the device to be retracted through the abdominal wall. The raised portion 2047 of the main shaft 2040 provides an abutment surface such that they define the home position for the deployment levers. As the device is removed the suture tails are pulled through the central hole 2045 until the device is external to the abdomen, leaving the suture tails 255 exposed on the abdominal wall. The user then uses the suture tails to effect a closure of the defect by placing a knot in the suture as previously describe for Figure 1B.

In the configuration of Figures 26 to 28 the entire device is moved relative to the abdominal wall. It will be appreciated that in other configurations that the device may be configured to incorporate both rotational and axial relative movement of the needle tips to its shield. Similarly to that described with Figures 26 to 28, the rotational relative movement allows for a rotational movement of the needle tips relative to the shield to facilitate movement of the needle tips out and away from their housed configuration. A subsequent longitudinal or axial relative movement- or movement parallel to a longitudinal axis of the shield - of the needle end portions may facilitate the active driving of the needle tips into the abdominal wall to facilitate a disengagement of the anchor from the needle tip and its delivery into the abdominal wall.

The anchor described heretofore has been described with reference to attachment of suture about an external surface of the anchor. Figure 29 shows a variation to this configuration whereby an anchor 3251 comprises a central lumen 3076 though which a needle tip may pass. It also features a hole (not shown) formed in a side surface of the body of the anchor and providing communication to an interior volume of the anchor. In this configuration suture may be passed along the central lumen 3076 and a side slot 3066 defined therein. The suture enters into the body of the anchor and can then be passed outwardly through the hole and a knot 3056 formed at the end of the suture to secure the end of the suture relative to the anchor. An advantage of this configuration is that the knot 3056 is bulky and is external to the lumen of the anchor, thereby allowing the needle passage thorough the anchor. The slot 3066 provides a break in the side wall of the anchor and will allow the suture to follow side walls of the slot to adopt a T-Bar configuration such as shown in Figure 29B.

Figure 30 shows a modification to a deployment device whereby each drive wire 2075 is configured to simultaneously receive and retain a plurality of anchors 251A, 251B,. In the example of Figure 30, the drive wire 2075 is shown independently of the shield housing. Certain preferred configurations will locate the drive wire within a shield such as that described with reference to Figure 26. However the use of a single driver wire that may be used to simultaneously deliver a plurality of individual anchors into the abdominal cavity for subsequent placement into the abdominal wall and may, in certain configurations, omit the use of a shield. In order to facilitate the seating of a plurality of anchors 251, the length of the step-down portion 2051 that was described with reference to Figure 27C may be extended to seat a plurality of anchors. As shown in Figure 30A, each anchor is configured such that a leading portion of a first anchor can be received within and provide a seat for a trailing portion of a second anchor. Each anchor is coupled to an individual piece of suture 255 such that each anchor 251 may be independently tensioned or secured relative to the drive wire 2075. In delivery, such as shown in Figure 30B the drive wire is driven upwardly into the abdominal wall so as to locate the outermost anchor 251A of the stack of anchors 251A, 251B, into the abdominal wall. On receipt in the abdominal wall and a loosening of the tension for that anchor 251A, that outermost anchor 251A will preferentially separate from the stack and remain in the abdominal wall when the driver wire 2075 is pulled back- as shown in Figure 30C. The process can be continued for the other anchors in the stack.

Although this embodiment shows a stack of two anchors it will be understood that this is not intended to be limiting and that a number of anchors could be configured this way once the length of the step-down portion 3051 is adjusted accordingly.

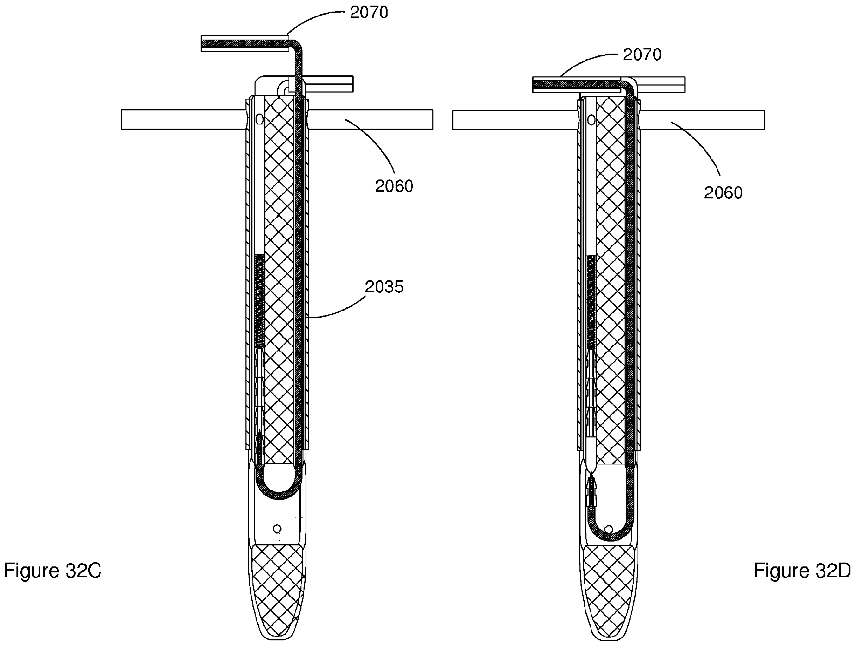

Figures 31 and 32 show a modification to the arrangement of Figure 30 whereby the device is again configured to transport a plurality of anchors simultaneously into the abdominal cavity wherein they can be independently deployed or delivered into the abdominal wall. In this configuration the device is however configured to transport the anchors within the shield as opposed to a plurality of anchors being simultaneously seated upon the same drive wire 2075.

In this configuration a stack 3200 of individual anchors are arranged within the shield main shaft. Similarly to the channel 2043 that was described with reference to Figure 27B for locating the drive wire 2075, the plurality of anchors may be seated within an anchor channel that is formed in the shaft main body. This defines a cassette for retention of the anchors until they are presented onto the tip. The anchors may be spring loaded by means of a spring 3201 that will effect a positive bias on the anchors towards the tip portion of the device. The anchors will be retained within the channel until such time as the needle tip 2051 displaces a deployment mechanism which delivers the lowest anchor within the stack onto the step-down portion 2051. In this way movement of the tip effects a discharge of the anchors from their cassette. Subsequent rotation of the needle end portion with its located anchor 251 out and away from the shield allows the delivery of that anchor into the abdominal wall. Subsequent rotation back into alignment with the anchor channel facilitates a reloading of the drive wire 2075 with another anchor. It will be appreciated from inspection of relative location of the driver wire handle portion 2070 with the handle 2060 of the shield in Figures 32C and 32D, that this reloading may be effected by relative movement in an axial direction of the driver wire 2075 relative to the shield 2035.

Figure 33A and 33B show a further modification to a shielded anchor delivery system. In this configuration which shares many features to that described before, a shield 2035 is provided to house or shield needle drivers during a delivery of the needle drivers into and out of the abdominal cavity. The needle drivers are dimensioned to accommodate individual anchors at a tip portion of the needle driver. On location within the abdominal cavity, the needle drivers are moveable out and away from the shield so as to expose the anchors external of the shield. This device also features a lumen 3306 which runs through the entire length of the device. The function of this lumen is to receive a camera system and the lumen is dimensioned to receive a camera. The lumen may be provided with a valve or other sealing mechanism to facilitate the closure of the lumen on occasions where the camera is not present.. It will be appreciated that the device could be presented in another configuration where this channel is not present, and accordingly the overall profile of the device can be smaller.

Where Figure 26 details such movement as being effected through a manual rotation of a handle of a drive wire through an arcuate motion effecting a direct and corresponding movement of the end of the drive wire and seated anchor, in Figure 33, the movement is effect through a gearing mechanism. A first cog arrangement 3300 is located with the shield and is coupled to an actuator 3305 that in use is located externally of the abdominal cavity. Rotation of the actuator 3305 effects a corresponding movement of the cog that is located internally of the abdominal cavity. This cog is interleaved with at least one secondary cog that is coupled to the anchor delivery wire 2075. By effecting a rotation of the actuator the anchor delivery wire can be moved into and out of a deployment configuration. The geared coupling of the tip to the shield effects and allows a controlled deployment.

As shown in Figure 33, the shield in this configuration comprises first 3302A and second 3302B parts. The first and second parts define a window there between through which the tips may move when travelling between the shielded and deployed configuration. The dimensions of the window are determined by the distance between the first and second parts. While certain configurations may provide that distance as a fixed distance, other configurations of a device provided in accordance with the present teaching may provide for a movement of the first and second parts relative to one another so as to allow for a closure of the window during periods when it is desired not to allow access to the tips and their associated anchors.

Figure 34 shows a sequence of views of one such configuration. In this arrangement the first 3302A and second 3302B parts are movable between an open and a closed configuration. In a closed configuration such as shown in Figure 34A the first and second parts abut against one another. This may be usefully employed during the passage of the device through the abdominal wall. Once the first part 3302A has passed successfully through the abdominal wall-as shown in Figure 34B, the parts can be moved away from one another to define the window through which the need tip(s) 2052 can move in their arcuate path out and away from the shield to adopt the deployed configuration - Figure 34C. The device is then retracted back towards the abdominal wall to facilitate the presentation and delivery of the anchors into the abdominal wall - Figure 34D. The subsequent pushing of the device back into the abdominal cavity effects a discharge of the anchors from the tip. This discharge arises from the fact that the friction resultant from contact of the anchor surface with the abdominal wall is greater than that friction in the contact between the anchor delivery tip such that once the device moves away from the abdominal wall- as shown in Figure 34E the anchors will preferentially stay in the abdominal wall. The first and second parts can then be moved again to adopt a closed configuration - Figure 34F - and the entire device retracted from the cavity.

While now shown in Figure 34, this entire sequence could be visualised by a surgeon through passing a camera through the lumen 3306.

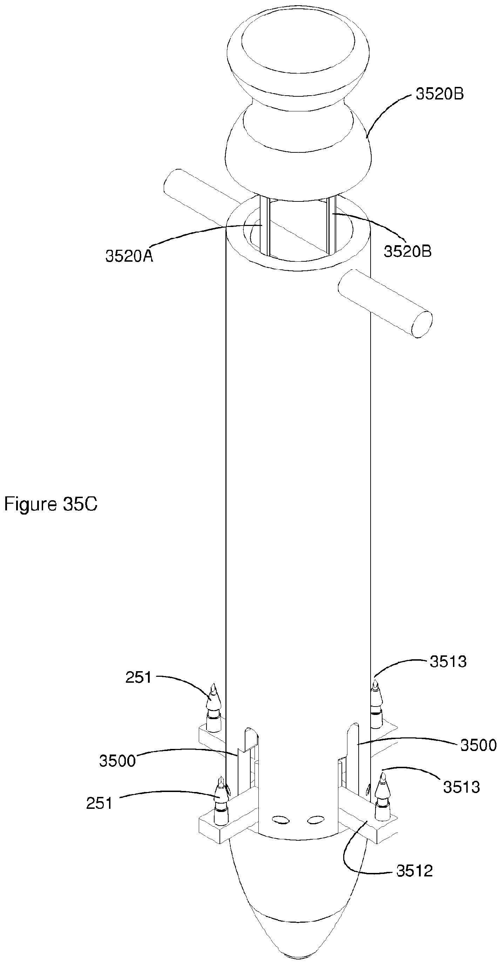

Figure 35 show another arrangement whereby the first 3302A and second 3302B parts are not moveable relative to one another, and instead of a single window 3303 (Fig 34B) extending circumferentially about the surface of the shield, individual windows 3500 are formed for each of the anchor delivery tips 3513. In this configuration, each tip has its own distinct window that it will pass through when passing from the shielded configuration to the deployed configuration. In this configuration and similarly to that previously described, each anchor delivery tip is formed on a shaft with a distal end and a proximal end. The curved portion 3510 in this configuration is a perpendicular curve such that the tip 3513 formed on the distal end 3511 is perpendicular to the proximal end 3512. In this configuration the tip pivots relative to the shield between the shielded configuration and the deployed configuration. In the shielded configuration- Figure 35A - a lower surface 3512A of the proximal end 3512 provides a closure member for the window 3500. This is achieved by pivoting the proximal portion through 90 degrees such that it is substantially parallel with a longitudinal axis of the shield body. In the deployed configuration the lower surface 3512A- shown in Figure 35B- is substantially transverse to the longitudinal axis of the shield body.

To facilitate this movement between the shielded and deployed configurations-it is possible to provide an actuation member 3520 -as shown in Figures 35C and 36. In this exemplary arrangement, the actuation member 3520 comprises first 3520A and second 3520B shafts which couple a handle 3530 of the device to the anchor delivery needles. The handle 3530 is moveable relative to the body of the shield and this movement effects a corresponding movement-through the actuation member 3520 - of the anchor delivery needles. In this exemplary arrangement the anchor delivery needle(s) are configured to pivot relative to the shield body. The actuation members 3520A, 3520B are coupled to moveable slide 3540 whose travel effects the pivoting of the anchor delivery needles into and out of the shield. This movement is such that when in the shielded configuration, anchors provided on needles on opposing sides of the device are orientated to face one another- as shown in Figure 36A. While the needle pivots about a point in a direction that is perpendicular to the longitudinal axis of the shield, the movement of the needle and anchor is along an arcuate path that is in a plane that is parallel to the longitudinal axis of the shield.

Another actuation mechanism that could facilitate movement along an arcuate path that in a plane that is parallel to the longitudinal axis of the shield could be effected by having the anchors provided on needles on opposing sides of the device being initially orientated to face away from one another in the shielded configuration. A path- similar in concept to a ski jump- would be defined within the shield. Initially in the shielded configuration, the needle delivery tips are retained towards the top of the sloped path and when biased outwardly by an actuation member will travel along the path and outwardly of the shield body. Desirably in this configuration and that described with reference to Figure 35, the length of travel defined by the handle 3530 relative to the top of the shield body corresponds to the necessary movement required to move from the shielded and deployed configuration.

Devices which have been disclosed here and deploy four anchors simultaneously may present difficulties to determine after deployment of the anchors the actual location of the anchors- for example it may be difficult to determine which anchors are placed opposite to each other based on the exposed suture on the exterior or the abdomen. This problem can be overcome by providing suture of differing colour for each pair. Further to this there may need to be specific instructions depending on the incision length where suture may need to be tied in an "X" configuration or parallel across a defect. In such an instance it may be advantageous to provide each strand of suture in a different colour such that either of the two tying configuration could be achieved.

It will also be appreciated that for the aforementioned embodiments which describe the deployment of two anchors have been used, any number of anchors may be deployed, for example where a large port needs to be closed. For these larger ports, an insufflation port would need to be included to facilitate pneumoperitoneum. Additionally the device as herein described may be used to deliver the initial anchoring sutures of a Hasson procedure. The suture portion of the anchors can be affixed to the Hasson sleeve of choice.

While preferred arrangements have been described in an effort to assist in an understanding of the teaching of the present invention it will be appreciated that it is not intended to limit the present teaching to that described and modifications can be made without departing from the scope of the invention.

It will be appreciated that the exemplary arrangements or examples of devices have been described with reference to the Figures attached hereto. Where a feature or element is described with reference to one Figure, it will be understood that the feature or element could be used with or interchanged for features or elements described with reference to another Figure or example. The person of skill in the art, when reviewing the present teaching, will understand that it is not intended to limit the present teaching to the specifics of the illustrated exemplary arrangements as modifications can be made without departing from the scope of the present teaching.

The words comprises/comprising when used in this specification are to specify the presence of stated features, integers, steps or components but does not preclude the presence or addition of one or more other features, integers, steps, components or groups thereof.

## Claims

1. An anchor delivery device comprising:
a shaft (231) with a distal end (221) and a proximal end (241), the shaft having a curved portion (221) comprising a sharp tip at the distal end of the shaft wherein the curved portion (221) has a geometry such that the tip is orientated towards the proximal end of the shaft; and
wherein the tip (201) is configured to receive and deliver a removable anchor (251), the anchor being coupled to suture, the tip being dimensioned to be received through a received anchor, the device further comprising a shield (280), the shield (280) and tip being moveable relative to one another between a shielded configuration and a deployed configuration, and wherein movement between the shielded configuration and the deployed configuration is along an arcuate path and wherein in a deployed configuration the device is configured to operably present and deliver the anchor into an abdominal wall from inside an abdominal space.

2. The device of claim 1 wherein the arcuate path is in a plane transverse to a longitudinal axis of the shield or is in a plane co-linear with a longitudinal axis of the shaft.

3. The device of claim 1 or 2 wherein the shield and tip are rotatable relative to one another.

4. The device of any preceding claim wherein the tip is moveable relative to the shield, wherein the movement is a pivot movement or wherein the movement is a rotation.

5. The device of any preceding claim wherein the tip and shield are coupled to one another through a gear mechanism such that the movement can be controlled.

6. The device of any preceding claim wherein the shield defines a body of the device.

7. The device of any one of claims 1 to 5 wherein the shield is slideable relative to the shaft.

8. The device of any preceding claim wherein a suture is attached to the removable anchor or integrally formed with the removable anchor or the anchor comprises a flexible element extending from the anchor and wherein an eyelet or notch is provided to tension the suture towards the distal end of the shaft.

9. The device of any one of claims 1 to 8 wherein shaft and tip are hollow and device is configured to have multiple anchors loaded within the shaft and tip.

10. The device of any preceding claim comprising first and second anchors.

11. The device of claim 10 wherein the shield comprises a cassette dimensioned to receive a plurality of anchors, the tip being moveable to engage with an anchor provided within the cassette to allow a loading of the tip with an anchor from the cassette.

12. The device of any preceding claim comprising a plurality of tips which are each moveable relative to the shield to allow concurrent deployment of anchors from different sides of the device.

13. A wound closure device comprising an anchor delivery device as claimed in any preceding claim and further comprising a bioabsorable closure surface configured to be located and retained within a surgical site during a wound repair, the closure surface comprising at least a first and second suture engaging portion configured to receive at least a first and second suture coupled to first and second anchors respectively, the suture being operably tensioned against the closure surface through a location of the first and second anchors within the abdominal wall to effect a retention of the closure surface in situ within the wound.

14. The device of claim 13 wherein the closure surface is formed from a flexible membrane or mesh.

15. The device of claims 14 wherein the closure surface is foldable to assist in delivery through a wound formed in the abdominal wall.

## Patentansprüche

1. Ankerzuführvorrichtung, umfassend:
einen Schaft (231) mit einem distalen Ende (221) und einem proximalen Ende (241), wobei der Schaft einen gekrümmten Abschnitt (221), der eine scharfe Spitze umfasst, am distalen Ende des Schafts aufweist, wobei der gekrümmte Abschnitt (221) eine solche Geometrie aufweist, dass die Spitze in Richtung des proximalen Endes des Schafts gerichtet ist; und
wobei die Spitze (201) zum Aufnehmen und Zuführen eines entfernbaren Ankers (251) konfiguriert ist, wobei der Anker mit Nahtmaterial verbunden ist, die Spitze so dimensioniert ist, dass sie durch einen aufgenommenen Anker aufgenommen wird und die Vorrichtung ferner einen Schutz (280) umfasst, wobei der Schutz (280) und die Spitze zueinander zwischen einer geschützten Konfiguration und einer ausgefahrenen Konfiguration beweglich sind, und wobei die Bewegung zwischen der geschützten Konfiguration und der ausgefahrenen Konfiguration entlang eines bogenförmigen Pfads verläuft, und wobei die Vorrichtung in einer ausgefahrenen Konfiguration so konfiguriert ist, dass sie den Anker betriebsbereit in eine Bauchwand vom Inneren eines Bauchraums führt und freisetzt.

2. Vorrichtung nach Anspruch 1, wobei sich der bogenförmige Pfad auf einer Ebene befindet, die quer zu einer Längsachse des Schutzes verläuft, oder sich auf einer Ebene befindet, die kollinear zu einer Längsachse des Schafts verläuft.

3. Vorrichtung nach Anspruch 1 oder 2, wobei der Schutz und die Spitze zueinander drehbar sind.

4. Vorrichtung nach einem der vorhergehenden Ansprüche, wobei die Spitze bezüglich des Schutzes beweglich ist, wobei die Bewegung eine Schwenkbewegung ist, oder wobei die Bewegung eine Drehung ist.

5. Vorrichtung nach einem der vorhergehenden Ansprüche, wobei die Spitze und der Schutz miteinander über ein Getriebe verbunden sind, sodass die Bewegung gesteuert werden kann.

6. Vorrichtung nach einem der vorhergehenden Ansprüche, wobei der Schutz einen Körper der Vorrichtung ausmacht.

7. Vorrichtung nach einem der Ansprüche 1 bis 5, wobei der Schutz bezüglich des Schafts verschiebbar ist.

8. Vorrichtung nach einem der vorhergehenden Ansprüche, wobei das Nahtmaterial an dem entfernbaren Anker befestigt ist oder einstückig mit dem entfernbaren Anker ausgebildet ist, oder der entfernbare Anker ein flexibles Element umfasst, das sich vom Anker erstreckt und wobei eine Öse oder eine Kerbe vorgesehen ist, um das Nahtmaterial in Richtung des distalen Endes des Schafts zu spannen.

9. Vorrichtung nach einem der Ansprüche 1 bis 8, wobei der Schaft und die Spitze hohl sind und die Vorrichtung so konfiguriert ist, dass sie mehrere Anker aufweist, die in den Schaft und die Spitze geladen sind.

10. Vorrichtung nach einem der vorhergehenden Ansprüche, die erste und zweite Anker umfasst.

11. Vorrichtung nach Anspruch 10, wobei der Schutz eine Kassette umfasst, die zum Aufnehmen mehrerer Anker dimensioniert ist, wobei die Spitze beweglich ist, um mit einen in der Kassette vorgesehen Anker in Eingriff zu kommen, um ein Beladen der Spitze mit einem Anker aus der Kassette zu ermöglichen.

12. Vorrichtung nach einem der vorhergehenden Ansprüche, die mehrere Spitzen umfasst, die jeweils bezüglich des Schutzes beweglich sind, um ein gleichzeitiges Ausfahren von Ankern von verschiedenen Seiten der Vorrichtung zu ermöglichen.

13. Wundverschlussvorrichtung, die eine Ankerzuführungsvorrichtung nach einem der vorhergehenden Ansprüche umfasst und ferner eine bioresorbierbare Verschlussoberfläche umfasst, die zum Anbringen und Zurückhalten innerhalb einer chirurgischen Eingriffsstelle während eines Wundverschlusses konfiguriert ist, wobei die Verschlussoberfläche mindestens einen ersten und zweiten mit Nahtmaterial in Eingriff kommenden Abschnitt umfasst, um mindestens ein erstes und zweites Nahtmaterial aufzunehmen, das mit dem ersten bzw. dem zweiten Anker verbunden ist, wobei das Nahtmaterial betriebswirksam gegen die Verschlussoberfläche durch einen Ort des ersten und zweiten Ankers in der Bauchwand gespannt ist, um ein Zurückhalten der Verschlussoberfläche in situ in der Wunde zu bewirken.

14. Vorrichtung nach Anspruch 13 wobei die Verschlussoberfläche aus einer flexiblen Membran oder Netz ausgebildet ist.

15. Vorrichtung nach Anspruch 14 wobei die Verschlussoberfläche faltbar ist, um beim Einführen durch eine Wunde zu helfen, die in der Bauchwand ausgebildet ist.

## Revendications

1. Dispositif de distribution d'élément d'ancrage comprenant :
une tige (231) avec une extrémité distale (221) et une extrémité proximale (241), la tige ayant une partie incurvée (221) comprenant une pointe acérée au niveau de l'extrémité distale de la tige dans lequel la partie incurvée (221) a une géométrie de sorte que la pointe est orientée vers l'extrémité proximale de la tige ; et
dans lequel la pointe (201) est configurée pour recevoir et distribuer un élément d'ancrage amovible (251), l'élément d'ancrage étant couplé à la suture, la pointe étant dimensionnée pour être reçue à travers un élément d'ancrage reçu, le dispositif comprenant en outre une protection (280), la protection (280) et la pointe pouvant se déplacer l'une par rapport à l'autre entre une configuration protégée et une configuration déployée, et dans lequel le mouvement entre la configuration protégée et la configuration déployée se fait le long d'un chemin arqué et dans lequel dans une configuration déployée, le dispositif est configuré pour présenter et distribuer de manière opérationnelle l'élément d'ancrage dans une paroi abdominale depuis l'intérieur d'un espace abdominal.

2. Dispositif selon la revendication 1, dans lequel le chemin arqué se trouve dans un plan transversal à un axe longitudinal de la protection ou se trouve dans un plan colinéaire avec un axe longitudinal de la tige.

3. Dispositif selon la revendication 1 ou 2, dans lequel la protection et la pointe peuvent tourner l'une par rapport à l'autre.

4. Dispositif selon une quelconque revendication précédente, dans lequel la pointe est mobile par rapport à la protection, dans lequel le mouvement est un mouvement de pivotement ou dans lequel le mouvement est une rotation.

5. Dispositif selon une quelconque revendication précédente, dans lequel la pointe et la protection sont couplées l'une à l'autre par l'intermédiaire d'un mécanisme d'engrenage de sorte que le mouvement peut être commandé.

6. Dispositif selon une quelconque revendication précédente, dans lequel la protection définit un corps du dispositif.

7. Dispositif selon l'une quelconque des revendications 1 à 5, dans lequel la protection peut coulisser par rapport à la tige.

8. Dispositif selon une quelconque revendication précédente, dans lequel une suture est fixée à l'élément d'ancrage amovible ou intégralement formée avec l'élément d'ancrage amovible ou l'élément d'ancrage comprend un élément flexible s'étendant depuis l'élément d'ancrage et dans lequel un œillet ou une encoche est prévu(e) pour tendre la suture vers l'extrémité distale de la tige.

9. Dispositif selon l'une quelconque des revendications 1 à 8, dans lequel la tige et la pointe sont creuses et le dispositif est configuré pour avoir plusieurs éléments d'ancrage chargés à l'intérieur de la tige et de la pointe.

10. Dispositif selon une quelconque revendication précédente, comprenant des premier et second éléments d'ancrage.

11. Dispositif selon la revendication 10, dans lequel la protection comprend une cassette dimensionnée pour recevoir une pluralité d'éléments d'ancrage, la pointe étant mobile pour venir en prise avec un élément d'ancrage prévu à l'intérieur de la cassette afin de permettre un chargement de la pointe avec un élément d'ancrage depuis la cassette.

12. Dispositif selon une quelconque revendication précédente, comprenant une pluralité de pointes qui sont chacune mobiles par rapport à la protection pour permettre le déploiement simultané d'éléments d'ancrage depuis différents côtés du dispositif.

13. Dispositif de fermeture de plaie comprenant un dispositif de distribution d'élément d'ancrage selon une quelconque revendication précédente et comprenant en outre une surface de fermeture bioabsorbable configurée pour être localisée et retenue à l'intérieur d'un site chirurgical pendant une réparation de plaie, la surface de fermeture comprenant au moins une première et une seconde parties de mise en prise de suture configurées pour recevoir au moins une première et une seconde sutures couplées aux premier et second éléments d'ancrage respectivement, la suture étant tendue de manière opérationnelle contre la surface de fermeture à travers un emplacement des premier et second éléments d'ancrage à l'intérieur de la paroi abdominale pour effectuer une rétention de la surface de fermeture in situ à l'intérieur la plaie.

14. Dispositif selon la revendication 13, dans lequel la surface de fermeture est formée depuis une membrane ou un maillage flexibles.

15. Dispositif selon la revendication 14, dans lequel la surface de fermeture est pliable pour faciliter la distribution à travers une plaie formée dans la paroi abdominale.
